# NEUE EUROPÄISCHE PATENTSCHRIFT

(11) **EP 1 322 592 B2**
(45) Veröffentlichungstag und Bekanntmachung des Hinweises auf die Entscheidung über den Einspruch: **17.06.2009**
(45) Hinweis auf die Patenterteilung: 28.07.2004
(21) Anmeldenummer: 01969791.1
(22) Anmeldetag: 28.09.2001
(51) Int. Cl.: C07C 217/74, C07C 215/64, C07C 215/42, A61K 31/35, A61P 25/00

(54) **SUBSTITUIERTE 1-AMINOBUTAN-3-OL-DERIVATE**
SUBSTITUTED 1-AMINOBUTAN-3-OL DERIVATIVES
DERIVES DE 1-AMINOBUTAN-3-OL SUBSTITUES

(30) Priorität: 29.09.2000 DE 10049483
(43) Veröffentlichungstag der Anmeldung: 02.07.2003
(73) Patentinhaber: Grünenthal GmbH, 52078 Aachen (DE)
(72) Erfinder: BUSCHMANN, Helmut, 08950 Esplugues de Llobregat (ES); MAUL, Corinna, 52066 Aachen (DE); SUNDERMANN, Bernd, 52066 Aachen (DE); JAGUSCH, Utz-Peter, 52066 Aachen (DE); HAURAND, Michael, 52078 Aachen (DE); CHIZH, Boris, Cambridge CB2 4NW (GB)
(86) Internationale Anmeldenummer: PCT/EP2001/011231
(87) Internationale Veröffentlichungsnummer: WO 2002/028817

(56) Entgegenhaltungen:
- EP-A1- 0 799 819
- US-A- 4 155 935
- DATABASE CA [Online] CHEMICAL ABSTRACTS SERVICE, COLUMBUS, OHIO, US; KAUFFMANN, THOMAS ET AL: "Alkylchromium and alkylmanganese reagents. IV. The aldehyde-selective and cheleselective alkylation of organic carbonyl compounds with monoalkylchromium(III) reagents" retrieved from STN Database accession no. 116:83175 XP002184288 & CHEM. BER. (1992), 125(1), 157-62 ,
- MOELM D ET AL: "FRAGMENTATION REACTIONS OF QUATERNIZED GAMMA-AMINO ALCOHOLS - DIASTEREOSELECTIVE SYNTHESIS OF HIGHLY FUNCTIONALIZED OXETANES AND UNSATURATED ALDEHYDES AND KETONES WITH A (Z)-C-C DOUBLE BOND" EUROPEAN JOURNAL OF ORGANIC CHEMISTRY, WILEY-VCH VERLAG, WEINHEIM, DE, Bd. 10, Nr. 10, 1998, Seiten 2185-2191, XP000993116 ISSN: 1434-193X
- J. S. BRYANS ET AL: '3-Substituted GABA Analogs with Central Nervous System Activity: A Review', 1999, JOHN WILEY & SONS, INC. Seiten 149 - 177
- YUKINORI NAGAKURA ET AL: 'Allodynia and Hyperalgesia in Adjuvant-Induced Arthritic Rats : Time Course of Progression and Efficacy of Analgesics' THE JOURNAL OF PHARMACOLOGY AND EXPERIMENTAL THERAPEUTICS Bd. 306, Nr. 2, 2003, US, Seiten 490 - 497
- K. FLICK ET AL: 'Untersuchungen zur chemischen Struktur und analgetischen Wirkung von phenylsubstituierten Aminomethylcyclohexanolen' ARZNEIMITTEL-FORSCHUNG / DRUG RESEARCH Bd. 28, Nr. 1, 1978, Seiten 107 - 113
- B. A. CHIZH ET AL: 'Antinociception and (R,S)-alpha-amino-3-hydroxy-5-methyl-4-isox azole propionic acid antagonism by gabapentin in the rat spinal cord in vivo' NAUMYN-SCHMIEDEBERG'S ARCH PHARMACOL Bd. 362, 2000, Seiten 197 - 200

## Beschreibung

Die vorliegende Erfindung betrifft substituierte 1-Aminobutan-3-ol-Derivate, Verfahren zu deren Herstellung, Arzneimittel enthaltend diese Verbindungen und die Verwendung von substituierten 1-Aminobutan-3-ol-Derivaten zur Herstellung von Arzneimitteln.

Das cyclische GABA Analoge Gabapentin ist ein klinisch erprobtes Antiepileptikum. Gabapentin zeigt zudem weitere interessante, medizinische relevante Eigenschaften, insbesondere als Analgetikum. Interessant sind deshalb neue Strukturklassen, die Affinität zur Gabapentin-Bindungsstelle aufweisen. Es besteht bei den genannten Indikationen weiterer Bedarf an Substanzen, die in ihren Eigenschaften Übereinstimmungen mit Gabapentin zeigen, beispielsweise in der analgetischen Wirkung.

US Patent Nr 4 155 935 offenbart cis-2-(Dimethylamino-m-substituierte-Benzyl)-Cyclohexanol verbindungen, die analgetische Wirkungen aufweisen.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Aufgabe der Erfindung war es daher, neue Strukturen, die Affinität zur Gabapentin-Bindungsstelle und/oder entsprechende physiologische Wirksamkeiten, beispielsweise in Hinblick auf Analgesie, aufweisen, aufzufinden.

Gegenstand der Erfindung sind daher substituierte 1-Aminobutan-3-ol-Derivate der allgemeinen Formel I, 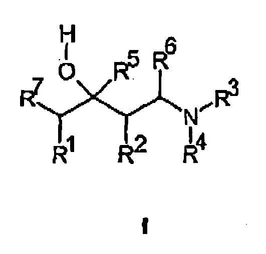 , worin
R¹ und R² zusammen einen (CH₂)₂₋₉-Ring bilden, der gegebenfalls mit C₁₋₈-Alkyl, verzweigt oder unverzweigt, gesättigt oder ungesättigt, unsubstituiert oder ein- oder mehrfach substituiert; oder Aryl, unsubstituiert oder ein- oder mehrfach substituiert; substituiert sein kann,
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; C₃₋₆-Cycloalkyl, gesättigt oder ungesättigt, unsubstituiert oder ein- oder mehrfach substituiert; Phenyl, Benzyl oder Phenethyl, unsubstituiert oder ein- oder mehrfach substituiert oder
die Reste R³ und R⁴ zusammen einen Ring bilden und CH₂CH₂OCH₂CH₂, CH₂CH₂NR²²CH₂CH₂ oder (CH₂)₃₋₆ bedeuten,
mit R²² ausgewählt aus H; C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl, gesättigt oder ungesättigt, gebundenem Aryl, C₃₋₁₀-Cycloalkyl oder Heteroaryl, jeweils einfach oder mehrfach substituiert oder unsubstituiert;
R⁵ ausgewählt ist aus C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt; Aryl, Heteroaryl, über gesättigtes oder ungesättigtes C₁₋₃-Alkyl gebundenem Aryl, über gesättigtes oder ungesättigtes C₁₋₃-Alkyl gebundenem C₃₋₁₀-Cycloalkyl oder über gesättigtes oder ungesättigtes C₁₋₃-Alkyl gebundenem Heteroaryl, wobei alle Aryl, Heteroaryl und Cycloalkyl-Reste jeweils unabhängig voneinander unsubstituiert oder einfach oder mehrfach substituiert sein können mit Resten unabhängig voneinander ausgewählt aus
F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
mit R¹⁸ ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
R¹⁹ und R²⁰ unabhängig voneinander ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
oder R¹⁹ und R²⁰ zusammen CH₂CH₂OCH₂CH₂, CH₂CH₂NR²¹CH₂CH₂ oder (CH₂)₃₋₆ bilden,
mit R²¹ ausgewählt aus H, C₁₋₁₀-Alkyl gesättigt oder ungesättigt, verzweigt oder unverzweigt, unsubstituiert oder einfach oder mehrfach substituiert;
R⁶ ausgewählt ist aus H; Aryl oder Heteroaryl; jeweils unsubstituiert oder ein- oder mehrfach substituiert; und
R⁷ ausgewählt ist aus Halogen, CF₃; C₃-C₉-Cycloalkyl, gesättigt oder ungesättigt, unsubstituiert oder ein- oder mehrfach substituiert; Aryl, Heteroaryl, jeweils unsubstituiert oder ein- oder mehrfach substituiert; über gesättigtes oder ungesättigtes C₁₋₃-Alkyl gebundenem Aryl, C₃₋₉-Cycloalkyl oder Heteroaryl, jeweils unsubstituiert oder ein- oder mehrfach substituiert
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Die erfindungsgemäßen Substanzen binden an die Gabapentin-Bindungsstelle und zeigen eine ausgeprägte analgetische Wirkung.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4-oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7-oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄-Cycloalkyl für C3-oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.
Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.
Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen, unter (CH₂)₁₋₄ ist -CH₂-, -CH₂-CH₂-, -CH₂-CH₂-CH₂- und -CH₂-CH₂-CH₂-CH₂- zu verstehen, etc.
Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.
Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5 ]thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert - wenn nicht ausdrücklich anders definiert - die Substitution des Aryls oder Heteroaryls mit R²³, OR²³ einem Halogen, vorzugsweise F und/oder Cl, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über C₁₋₃-Alkyl, gesättigt oder ungesättigt, oder eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über C₁₋₃-Alkyl, gesättigt oder ungesättigt, oder eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über C₁₋₃-Alkyl, gesättigt oder ungesättigt, oder eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter physiologisch verträgliche Salze mit Kationen oder Basen und physiologisch verträgliche Salze mit Anionen oder Säuren.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid-Salz.

In einer bevorzugten Ausführungsform der Erfindung ist bei den erfindungsgemäßen substituierten 1-Aminobutan-3-ol-Derivaten gemäß Formel I
R6 ausgewählt aus H oder Heteroaryl oder ist vorzugsweise ein Rest gemäß Formel II, 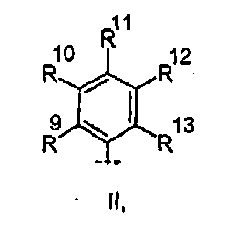 mit R⁹ bis R¹³, jeweils unabhängig voneinander ausgewählt aus H, F, Cl, Br, I, CF₃, OH, OR¹⁴, OCF₃, SR¹⁴, SO₂CH₃, SO₂CF_{3;} C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert; CN, COOR¹⁴, NO₂ oder
worin R⁹ und R¹⁰ oder R¹⁰ und R¹¹ zusammen einen OCH₂O- oder OCH₂CH₂O-Ring bilden, und
R¹⁴ ausgewählt ist aus C₁₋₆-Alkyl, verzweigt oder unverzweigt, gesättigt oder ungesättigt, unsubstituiert oder ein- oder mehrfach substituiert; Phenyl, Benzyl, Phenethyl oder Thiophenyl, jeweils unsubstituiert oder ein- oder mehrfach substituiert.
in einer weiteren, aber besonders bevorzugten Ausführungsform der Erfindung bilden bei den erfindungsgemäßen substituierten 1-Aminobutan-3-ol-Derivaten gemäß Formel I
R¹ und R² zusammen einen (CH₂)₂₋₅-Ring, der gegebenfalls mit C₁₋₆-Alkyl, verzweigt oder unverzweigt, gesättigt oder ungesättigt, unsubstituiert oder ein- oder mehrfach substituiert; oder Phenyl, unsubstituiert oder ein- oder mehrfach substituiert; substituiert sein kann, der aber vorzugsweise unsubstituiert ist.

In einer anderen bevorzugten Ausführungsform der Erfindung sind bei den erfindungsgemäßen substituierten 1-Aminobutan-3-ol-Derivaten gemäß Formel I
R³ und R⁴ jeweils unabhängig voneinander ausgewählt aus C₁₋₆-Alkyl, verzweigt oder unverzweigt, gesättigt oder ungesättigt, unsubstituiert oder ein- oder mehrfach substituiert. Vorzugsweise sind beide CH₃.
oder bilden die Reste R³ und R⁴ zusammen einen Ring und bedeuten CH₂CH₂NR²²CH₂CH₂ oder (CH₂)₃₋₆, bedeuten insbesondere zusammen oder (CH₂)₄₋₅ oder CH₂CH₂NR²²CH₂CH₂, mit R²² ausgewählt aus H oder C₁₋₆-Alkyl, gesättigt, verzweigt oder unverzweigt und unsubstituiert; insbesondere H oder CH₃.

In einer bevorzugten Ausführungsform der Erfindung ist bei den erfindungsgemäßen substituierten 1-Aminobutan-3-ol-Derivaten gemäß Formel I
R⁵ ausgewählt ist aus C₁₋₆-Alkyl, gesättigt oder ungesättigt, verzweigt oder unverzweigt, einfach oder mehrfach substituiert oder unsubstituiert; C₅₋₆-Cycloalkyl, Phenyl, Thiophenyl, Furyl, Benzofuranyl, Benzothiophenyl, Pyrrolyl, Pyridinyl, Pyrimidinyl, Chinolinyl, Isochinolinyl, Chinazolinyl, über gesättigtes oder ungesättigtes C₁₋₃-Alkyl gebundenem Phenyl, über gesättigtes oder ungesättigtes C₁₋₃-Alkyl gebundenem C₅₋₆-Cycloalkyl oder über gesättigtes oder ungesättigtes C₁₋₃-Alkyl gebundenem Thiophenyl, Furyl, Benzofuranyl, Benzothiophenyl, Pyrrolyl, Pyridinyl, Pyrimidinyl, Chinolinyl, Isochinolinyl, Chinazolinyl, wobei alle Aryl, Heteroaryl und Cycloalkyl-Reste jeweils unabhängig voneinander unsubstituiert oder einfach oder mehrfach substituiert sein können,
vorzugsweise
R⁵ ausgewählt ist aus Phenyl oder Thiophenyl, unsubstituiert oder einfach oder mehrfach, vorzugsweise mit F, Cl, Br, I, OH, O-C₁₋₄-Alkyl, C₁₋₆-Alkyl, NH₂ und/oder SH, substituiert; oder über gesättigtes oder ungesättigtes C₁₋₃-Alkyl gebundenem Phenyl, unsubstituiert oder einfach oder mehrfach, vorzugsweise mit F, Cl, Br, I, OH, O-C₁₋₄-Alkyl, C₁₋₆-Alkyl, NH₂ und/oder SH, substituiert,
insbesondere
R⁵ ausgewählt ist aus Phenyl unsubstituiert oder einfach oder mehrfach, vorzugsweise mit F, Cl, Br, I, OH, OCH₃, OC₂H₅, OC₃H₇, SH, CH₃, C₂H₅, C₃H₇ und/oder C₄H₉, substituiert.

Insgesamt ist es bezüglich des Substituenten R⁵ bevorzugt, wenn dann, wenn R⁵ ausgewählt ist aus direkt oder über gesättigtes oder ungesättigtes C1-3-Alkyl gebundenem Aryl, Heteroaryl oder Cycloalkyl, diese einfach oder mehrfach
vorzugsweise mit Resten unabhängig voneinander ausgewählt aus
F, Cl, Br, I, OR¹⁸, SR¹⁸, COOR¹⁸, NH₂; C₁₋₁₀-Alkyl, gesättigt oder ungesättigt, verzweigt oder unverzweigt, einfach oder mehrfach substituiert oder unsubstituiert;
mit R¹⁸ ausgewählt aus H; C₁₋₆-Alkyl, gesättigt oder ungesättigt, verzweigt oder unverzweigt, unsubstituiert oder einfach oder mehrfach substituiert;
insbesondere mit Resten unabhängig voneinander ausgewählt aus
F, Cl, Br, I, OH, O-C₁₋₄-Alkyl, C₁₋₆-Alkyl, NH₂ und/oder SH;
substituiert oder unsubstituiert sind.

In einer weiteren bevorzugten Ausführungsform der Erfindung ist bei den erfindungsgemäßen substituierten 1-Aminobutan-3-ol-Derivaten gemäß Formel **I**
R⁷ ausgewählt aus C₅-C₇-Cycloalkyl, gesättigt oder ungesättigt, unsubstituiert oder ein- oder mehrfach substituiert, vorzugsweise Cyclohexyl; Phenyl, unsubstituiert oder ein- oder mehrfach substituiert; über gesättigtes oder ungesättigtes C₁₋₃-Alkyl gebundenem Phenyl, jeweils unsubstituiert oder ein- oder mehrfach substituiert,
vorzugsweise R⁷ ausgewählt ist aus Phenyl, unsubstituiert oder ein- oder mehrfach substituiert; über gesättigtes oder ungesättigtes C₁₋₃-Alkyl gebundenem Phenyl, jeweils unsubstituiert oder ein- oder mehrfach substituiert.

In einer weiteren bevorzugten Ausführungsform der Erfindung ist bei den erfindungsgemäßen substituierten 1-Aminobutan-3-ol-Derivaten gemäß Formel I , wenn R5 und/oder R7 ausgewählt ist aus über gesättigtes oder ungesättigtes C₁₋₃-Alkyl gebundenem Aryl, C₃₋₉-Cycloalkyl oder Heteroaryl, das C₁₋₃-Alkyl über das Aryl, Heteroaryl oder Cycloalkyl gebunden ist, ausgewählt aus:
-CH₂-, -C₂H₄-, C₃H₆-, -C≡C-, -CH=CH-, -CH=CH-CH₂-, -CH₂-CH=CH-, -C≡C-CH₂- oder -CH2-C≡C-, vorzugsweise -CH₂-, -C₂H₄- oder -C≡C-.

In einer bevorzugten Ausführungsform der Erfindung sind die erfindungsgemäßen substituierten 1-Aminobutan-3-ol-Derivate ausgewählt aus der folgenden Gruppe:
- 2-Benzyl-1-(2,4-dichlor-benzyl)-6-dimethylaminomethyl-cyclohexanol
- 2-Benzyl-1-(3-chlor-benzyl)-6-dimethylaminomethyl-cyclohexanol
- 2-Benzyl-6-dimethylaminomethyl-1-(2,5-dimethyl-benzyl)-cyclohexanol
- 2-Benzyl-1-(2-chlor-6-fluor-benzyl)-6-dimethylaminomethyl-cyclohexanol
- 2-Benzyl-1-(4-chlor-benzyl)-6-dimethylaminomethyl-cyclohexanol
- 2-Benzyl-6-dimethylaminomethyl-1-(3-trifluormethyl-phenyl)-cyclohexanol
- 2-Benzyl-6-dimethylaminomethyl-1-(2-methyl-benzyl)-cyclohexanol
- 2-Benzyl-6-dimethylaminomethyl-1-(2-methoxy-phenyl)-cyclohexanol
- 2-Benzyl-1-(4-chlor-3-trifluormethyl-phenyl)-6-dimethylaminomethylcyclohexanol
- 2-Benzyl-1-(2-chlor-benzyl)-6-dimethylaminomethyl-cyclohexanol
- 2-Benzyl-1-(3,5-dichlor-phenyl)-6-dimethylaminomethyl-cyclohexanol
- 2-Benzyl-1-(3-chlor-phenyl)-6-dimethylaminomethyl-cyclohexanol
- 2-Benzyl-6-dimethylaminomethyl-1-(3-fluor-phenyl)-cyclohexanol
- 2-Benzyl-6-dimethylaminomethyl-1-(5-fluor-2-methoxy-phenyl)-cyclohexanol
- 2-Benzyl-1-cyclohexylmethyl-6-dimethylaminomethyl-cyclohexanol
- 2-Benzyl-6-dimethylaminomethyl-1-(4-methoxy-phenyl)-cyclohexanol
- 2-Benzyl-6-dimethylaminomethyl-1-p-tolyl-cyclohexanol
- 2-Benzyl-6-dimethylaminomethyl-1-(3-phenyl-propyl)-cyclohexanol
- 2-Benzyl-6-dimethylaminomethyl-1-(3-methoxy-phenyl)-cyclohexanol
- 2-Benzyl-6-dimethylaminomethyl-1-thiophen-2-yl-cyclohexanol
- 2-Benzyl-6-dimethylaminomethyl-1-phenylethynyl-cyclohexanol
- 2-Benzyl-6-dimethylaminomethyl-1-(4-fluor-phenyl)-cyclohexanol
- 2-Benzyl-6-dimethylaminomethyl-bicyclohexyl-1-ol
- 2-Benzyl-6-dimethylaminomethyl-1-m-tolyl-cyclohexanol
- 2-Benzyl-1-(4-tert-butyl-phenyl)-6-dimethylaminomethyl-cyclohexanol
- 2-Benzyl-6-dimethylaminomethyl-1-vinyl-cyclohexanol
- 2-Benzyl-6-dimethylaminomethyl-1-o-tolyl-cyclohexanol
- 2-Benzyl-6-dimethylaminomethyl-1-(4-fluor-3-methyl-phenyl)-cyclohexanol
- 1,2-Dibenzyl-6-dimethylaminomethyl-cyclohexanol
- 2-Benzyl-1-(4-chlor-phenyl)-6-dimethylaminomethyl-cyclohexanol
- 2-Benzyl-6-dimethylaminomethyl-1-phenyl-cyclohexanol
- 2-Dimethylaminomethyl-1-(2,5-dimethyl-phenyl)-6-(3-methoxy-phenyl)-cyclohexanol
- 1-Cyclohexylmethyl-2-dimethylaminomethyl-6-(3-methoxy-phenyl)-cyclohexanol
- 1-(2,4-Dichlor-benzyl)-2-dimethylaminomethyl-6-(3-methoxy-phenyl)-cyclohexanol
- 2-Dimethylaminomethyl-6-(3-methoxy-phenyl)-1 -phenylethynyl-cyclohexanol
- 2-Dimethylaminomethyl-6-(3-methoxy-phenyl)-1-o-tolyl-cyclohexanol
- 1-(4-Chlor-phenyl)-2-dimethylaminomethyl-6-(3-methoxy-phenyl)-cyclohexanol
- 2-Dimethylaminomethyl-1-(3-fluor-phenyl)-6-(3-methoxy-phenyl)-cyclohexanol
- 2-Dimethylaminomethyl-1-(3-fluor-4-methoxy-phenyl)-6-(3-methoxy-phenyl)-cyclohexanol
- 1-(3-Chlor-benzyl)-2-dimethylaminomethyl-6-(3-methoxy-phenyl)-cyclohexanol
- 2-Dimethylaminomethyl-1-(3-methoxy-benzyl)-6-(3-methoxy-phenyl)-cyclohexanol
- 1-(4-Chlor-3-trifluormethyl-phenyl)-2-dimethylaminomethyl-6-(3-methoxy-phenyl)-cyclohexanol
- 1-(3,5-Dichlor-benzyl)-2-dimethylaminomethyl-6-(3-methoxy-phenyl)-cyclohexanol
- 2-Dimethylaminomethyl-6-(3-methoxy-phenyl)-1-phenylethynyl-cyclohexanol
- 1-(4-Chlor-phenyl)-2-dimethylaminomethyl-6-(3-methoxy-phenyl)-cyclohexanol
- 2-Dimethylaminomethyl-6-(3-methoxy-phenyl)-1-o-tolyl-cyclohexanol
- 1-(3-Chlor-benzyl)-2-dimethylaminomethyl-6-(3-methoxy-phenyl)-cyclohexanol
- 2-Dimethylaminomethyl-1-(4-fluor-2-methoxy-phenyl)-6-(3-methoxy-phenyl)-cyclohexanol
- 2-Dimethylaminomethyl-1-(2-methoxy-phenyl)-6-(3-methoxy-phenyl)-cyclohexanol
- 2-Dimethylaminomethyl-1-(4-fluor-benzyl)-6-(3-methoxy-phenyl)-cyclohexanol
- 1-(3-Chlor-phenyl)-2-dimethylaminomethyl-6-(3-methoxy-phenyl)-cyclohexanol
- 2-Dimethylaminomethyl-6-(3-methoxy-phenyl)-1-p-tolyl-cyclohexanol
- 2-Dimethylaminomethyl-6-(3-methoxy-phenyl)-1-(3-phenyl-propyl)-cyclohexanol
- 2-Dimethylaminomethyl-1,6-bis-(3-methoxy-phenyl)-cyclohexanol
- 2-Dimethylaminomethyl-6-(3-methoxy-phenyl)-1-thiophen-2-yl-cyclohexanol
- 2-Dimethylaminomethyl-6-(3-methoxy-phenyl)-1-phenethyl-cyclohexanol
- 3-[3-Dimethylaminomethyl-2-(4-fluor-phenyl)-2-hydroxy-cyclohexyl]-phenol
- 3-(3-Dimethylaminomethyl-2-hydroxy-2-phenyl-cyclohexyl)-phenol
- 3-[2-(4-tert-Butyl-phenyl)-3-dimethylaminomethyl-2-hydroxy-cyclohexyl]-phenol
- 3-(3-Dimethylaminomethyl-2-hydroxy-2-vinyl-cyclohexyl)-phenol
- 1-(4-Chlor-phenyl)-2-dimethylaminomethyl-6-(3-methoxy-phenyl)-cyclohexanol
- 3-Dimethylaminomethyl-2-(3-methoxy-phenyl)-bicyclohexyl-2-ol
- 2-Benzyl-6-dimethylaminomethyl-1-(4-trifluormethyl-phenyl)-cyclohexanol
- 3-(2-Benzyl-6-dimethylaminomethyl-1-hydroxy-cyclohexyl)-phenol
- 3-(2-tert-Butyl-6-dimethylaminomethyl-1-hydroxy-cyclohexyl)-phenol
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate, insbesondere der Hydrochloridsalze.

Die erfindungsgemäßen Substanzen sind toxikologisch unbedenklich, so daß sie sich als pharmazeutischer Wirkstoff in Arzneimitteln eignen. Ein weiterer Gegenstand der Erfindung sind daher Arzneimittel enthaltend wenigstens ein substituiertes 1-Aminobutan-3-ol-Derivat, sowie gegebenenfalls geeignete Zusatz- und/oder Hilfsstoffe und/oder gegebenenfalls weitere Wirkstoffe, wie in den Ansprüchen 9-17 definiert.

Die erfindungsgemäßen Arzneimittel enthalten neben mindestens einem erfindungsgemäßen substituierten 1-Aminobutan-3-ol-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionsiösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte 1-Aminobutan-3-ol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten 1-Aminobutan-3-ol-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen substituierten 1-Aminobutan-3-ol-Derivats appliziert.

In einer bevorzugten Form des Arzneimittel liegt ein enthaltenes erfindungsgemäßes substituiertes 1-Aminobutan-3-ol-Derivat als reines Diastereomer und/oder Enantiomer, als Razemat oder als nicht-äquimolare oder äquimolare Mischung der Diastereomere und/oder Enantiomere vor.

Gabapentin ist ein bekanntes Antiepileptikum mit antikonvulsiver Wirkung. Neben dieser wird Gabapentin von auch in verschiedenen anderen Indikation eingesetzt, unter anderem von behandelnden Ärzten bei Migräne und bipolaren Störungen sowie Hitzewallungen (z.B. in der Postmenopause) verschrieben (M. Schrope, Modem Drug Discovery, September 2000, S. 11). Andere Indikationenen, in denen Gabapentin ein therapeutisches Potential zeigt, wurden während der Humanstudien und im klinischen Gebrauch identifiziert (J.S. Bryans, D.J. Wustrow; "3-Substituted GABA Analogs with Central Nervous System Activity: A Review" in Med. Res. Rev. (1999), S. 149-177). In diesem Übersichtsartikel wird detailliert die Wirkung von Gabapentin aufgelistet. So ist Gabapentin wirksam in der Behandlung chronischer Schmerzen und Verhaltensstörungen. Insbesondere sind aufgeführt: Antikonvulsive und antiepileptische Wirkungen, der Einsatz gegen chronischen, neuropathischen Schmerz, insbesondere thermische Hyperalgesie, mechanische Allodynie, Kälte-Allodynie. Weiter wirkt es gegen durch Nervenschädigungen ausgelöste Neuropathie, insbesondere eben neuropathischen Schmerz, wie auch inflammatorischen und postoperativen Schmerz erfolgreich. Gabapentin ist auch erfolgreich bei antipsychotischen Effekten insbesondere als Anxiolytikum. Weitere überprüfte Indikationen umfassen: Amyotropische Laterale Sklerose (ALS), Reflex Sympasthetic Dystrophy (RSD), Spastische Lähmung, Restless Leg Syndrom, Behandlung von Symptomen und Schmerz aufgrund von Multipler Sklerose, erworbener Nystagmus, Behandlung der Symptome der Parkinsonschen Krankheit, der schmerzvollen diabetischen Neuropathie und psychatrischer Störungen, z.B. bipolare Störungen, Stimmungsschwankungen, manisches Verhalten. Weiter erfolgreich war der Einsatz von Gabapentin bei erythromelalgischem Schmerz, postpoliomyelitisem Schmerz, trigeminaler Neuralgie und postherpetischer Neuralgie (Bryans und Wustrow (1999), a.a.O.). Allgemein bekannt und auch dem genannten Übersichtsartikel anhand der Beispiele zu entnehmen ist auch die allgemeine Wirksamkeit in neurodegenerativen Erkrankungen. Solche Neurodegenerativen Erkrankungen sind z.B. Morbus Alzheimer, Morbus Huntington, Morbus Parkinson und Epilepsie. Bekannt ist auch die Wirksamkeit von Gabapentin bei gastrointestinalen Schädigungen.

Alle erfindungsgemäßen Substanzen verdrängen Gabapentin von seiner - auch in der Wissenschaft bisher noch unbekannten - Bindungsstelle. Das impliziert aber, daß die erfindungsgemäßen Substanzen an der gleichen Bindungsstelle binden und über sie physiologische wirken werden, vermutlich mit dem gleichen Wirkungsprofil wie Gabapentin. Daß diese Annahme der gleichen Wirkung bei gleicher Bindungsstelle auch zutrifft, wird durch die analgetische Wirkung bewiesen. So verdrängen die erfindungsgemäßen Verbindungen nicht nur Gabapentin von seiner Bindungsstelle sondern wirken auch - wie Gabapentin - deutlich analgetisch.

Daher ist ein weiterer Gegenstand der Erfindung die Verwendung eines erfindungsgemäßen substituierten 1-Aminobutan-3-ol-Derivats zur Herstellung eines Arzneimittels zur Behandlung von Schmerz, insbesondere von neuropathischem, chronischem oder akutem Schmerz.

Die erfindungsgemäßen Substanzen sind auch zur Behandlung insbesondere mit neuropathischem Schmerz verbundener Symptome aber auch anderen verwandten Indikationen einsetzbar. Ein weiterer Gegenstand der Erfindung ist daher die Verwendung eines erfindungsgemäßen substituierten 1-Aminobutan-3-ol-Derivats zur Herstellung eines Arzneimittels zur Behandlung von Migräne, Hyperalgesie und Allodynie, insbesondere thermischer Hyperalgesie, mechanischer Hyperalgesie und Allodynie und Kälte-Allodynie, oder von inflammatorischem oder postoperativem Schmerz.

Auch in anderen Indikationen sind die erfindungsgemäßen Verbindungen einsetzbar. Daher ist ein weiterer Gegenstand der Erfindung die Verwendung eines erfindungsgemäßen substituierten 1-Aminobutan-3-ol-Derivats zur Herstellung eines Arzneimittels zur Behandlung von Epilepsie, Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

Dabei kann es bevorzugt sein, wenn ein verwendetes substituiertes 1-Aminobutan-3-o!-Derivat gemäß einem der Ansprüche 1 bis 11, als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung eines nichthumanen Säugetieres oder Menschen, das oder der eine Behandlung medizinisch relevanter Symptome benötigt, durch Verabreichung einer therapeutisch wiksamen Dosis eines erfindungsgemäßen substituierten 1-Aminobutan-3-ol-Derivats, oder eines erfindungsgemäßen Arzneimittels. Die Erfindung betrifft insbesondere entsprechende Verfahren zur Behandlung von Schmerz, insbesondere von neuropathischem, chronischem oder akutem Schmerz; Migräne, Hyperalgesie und Allodynie, insbesondere thermischer Hyperalgesie, mechanischer Hyperalgesie und Allodynie und Kälte-Allodynie, oder von inflammatorischem oder postoperativem Schmerz; Epilepsie, Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Alzheimer Disease, Huntington's Disease, Parkinson Disease und Epilepsie; von erythromelalgischem oder postpoliomyelitischem Schmerz, trigeminaler oder postherpetischer Neuralgie

Ein weiterer Gegenstand der Erfindung ist ein Verfahren zur Herstellung eines erfindungsgemäßen substituierten 1-Aminobutan-3-ol-Derivats wie in der folgenden Beschreibung und Beispielen ausgeführt.

### Allgemeine Herstellung der erfindungsgemäßen Verbindungen

Für die synthetischen Arbeiten sind in der Literatur beschriebene Reaktionen angewandt ( R.C.Larock, Comprehensive Organic Transformations, 2^{nd} edition, Wiley, New York 1999 und dort zitierte Literatur) sowie im Hause bekannte Erfahrungen eingebracht worden.

Substituierte 1-Aminobutan-3-ol-Derivate der allgemeinen Formel **I** lassen sich durch ein Verfahren herstellen, welches dadurch gekennzeichnet ist, daß man ein β-Aminoketon (im weiteren auch als Mannich-Basen bezeichnet) der Formel **Ia,** in der die Reste R¹ bis R⁴, R⁶ und R⁷ eine der oben für Formel **I** beschriebenen Bedeutungen haben, wobei diese Reaktion besonders bevorzugt ist für Verbindungen, in denen R⁶ ≠ H ist, 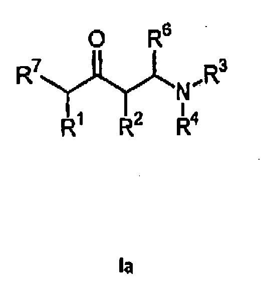 mit einer metallorganischen Verbindung der Formel **III**

R⁵-Z III

in der Z MgCl, MgBr, MgI oder Li bedeutet und R⁵ eine der oben für Formel **I** beschriebenen Bedeutungen hat, zu einer Verbindung der Formel **I** umsetzt.

Die Umsetzung eines β-Aminoketons **Ia** mit einer Grignardverbindung der Formel **III**, in der Z MgCl, MgBr oder Mgl bedeutet, oder mit einer lithiumorganischen Verbindung der Formel **III** kann in einem aliphatischen Ether, beispielsweise Diethylether und/oder Tetrahydrofuran, bei Temperaturen zwischen -70 °C und +60 °C durchgeführt werden. Lithiumorganische Verbindungen der Formel **III**, in der Z Cl, Br oder I bedeutet, lassen sich durch Umsetzung mit beispielsweise einer n-Butyllithium/Hexan-Lösung durch Halogen-Lithiumaustausch erhalten.

β-Aminoketone der allgemeinen Formel **Ia** lassen sich nach literaturbekannten Verfahren (Houben-Weyl - Methoden der Organischen Chemie, E21 b, 1995, S. 1925-1929; M. Tramontini, L. Angiolini, Mannich Bases, Chemistry and Uses,CRS Press, 1994 und dort zitierte Literatur) herstellen. Bevorzugt lassen sich β-Aminoketone der allgemeinen Formel **Ia** durch Umsetzung von Enaminen der allgemeinen Formel **IV**, wobei diese Reaktion besonders bevorzugt ist für Verbindungen, in denen R⁶ ≠ H ist, 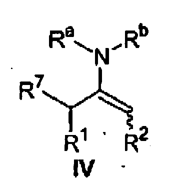 mit einem Iminiumsalz der allgemeinen Formel **V**, 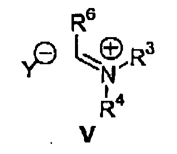 worin Y vorzugsweise Cl⁻, AlCl₄⁻, Br⁻ oder I⁻ bedeutet, erhalten.

Verbindungen, in denen R⁶ H entspricht, können analog mit den literaturbekannten Verfahren der klassischen Mannich-Reaktion über das Eschnmoser-Salz oder BuLi hergestellt werden.

Die Enamine der allgemeinen Formel **IV** werden nach literaturbekannten Verfahren durch die Umsetzung von Ketonen der allgemeinen Formel **VI** 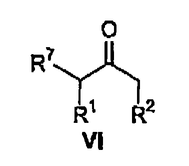 mit sekundären Amine, vorzugsweise Dimethylamin, Pyrrolidin, Piperidin oder Morpholin, erhalten. (Acta Chem. Scand. B 38, 1984, S. 49-53). Die Iminiumsalze der allgemeinen Formel V werden nach literaturbekannten Verfahren durch Umsetzung von Aminalen der allgemeinen Formel **VII** 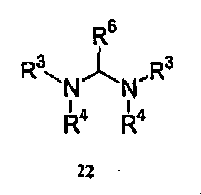 mit Säurechloriden, beispielsweise Acetylchlorid oder Thionylchlorid, hergestellt (Houben-Weyl-Methoden der Organischen Chemie, E21b, 1995, S. 1925-1929).

Die Imminiumsalze der allgemeinen Formel **V** müssen dabei nicht isoliert werden, sondern können in situ erzeugt und mit Enaminen der allgemeinen Formel **IV** zu Mannichbasen der allgemeinen Formel **Ia** umgesetzt werden (Angew. Chem. 106, 1994, S. 2531-2533). Aufgrund der Keto-Enol-Tautomerie analogen Enamin-Imin-Tautomerie sind statt der Enamine der allgemeinen Formel **IV** auch Imine der allgemeinen Formel **VIII** 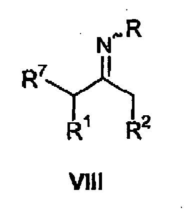 einsetzbar. Alternativ dazu können Ketone der allgemeinen Formel **VI** auch direkt mit Imminiumsalzen der allgemeinen Formel **V** umgesetzt werden.

Mannichbasen der allgemeinen Formel **Ia** können aber auch durch Umsetzung von Enaminen der allgemeinen Formel **IV** mit einem aromatischen oder heteroaromatischen Aldehyd der allgemeinen Formel **IX** 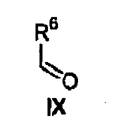 und einem sekundären Amin der allgemeinen Formel HNR³R⁴ **X** auch in Form des korrespondierenden Hydrochlorids HNR³R⁴·HCl, vorzugsweise in Gegenwart von Triethylamin, Chlortrimethylsilan und Natriumiodid direkt hergestellt werden (Synlett 1997, S. 974-976).

Die Mannichbasen der allgemeinen Formel **Ia** werden mit den oben beschriebenen Verfahren in Abhängigkeit von den Reaktionsbedingungen bevorzugt mit der relativen Konfiguration der allgemeine Formel **IIa** erhalten, 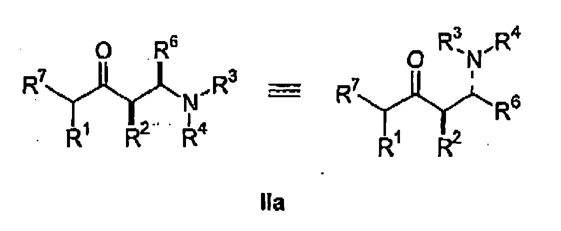 in denen die Aminogruppe *anti* zu R² angeordnet ist. Diese Verbindungen der allgemeinen Formel **IIa** lassen sich durch Kristallisation, auch ihrer Salze, beispielsweise der Hydrochloride, oder durch chromatographische Trennung diastereomerenrein erhalten.

Die Darstellung von Mannichbasen der allgemeinen Formel **Ia** durch 1,4-Addition sekundärer Amine der allgemeinen Formel **X** an Enone der allgemeinen Formel **XI,** 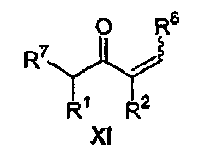 die aus der Aldolkondensation von Ketonen der allgemeinen Formel **VI** mit aromatischen oder heteroaromatischen Aldehyden der allgemeinen Formel **IX** erhalten werden, verläuft dagegen weniger stereoselektiv (US-Patent 4,017,637). Diese Vorgehensweise eignet sich daher zur Darstellung der anderen möglichen Stereosiomeren.

Werden chirale Amine zur Darstellung von Enaminen der allgemeinen Formel **IV** oder Imine der allgemeinen Formel **VIII** eingesetzt, so können in der nachfolgenden Mannichreaktion enantiomeren-angereicherte bis enantiomerenreine Mannichbasen der allgemeinen Formel **Ia** erhalten werden (Houben-Weyl - Methoden der Organischen Chemie, E21 b, 1995, S. 1925-1929).

Enantiomerenreine Mannichbasen der Formel **Ia** können auch durch eine Aminomethylierung unter Verwendung enantiomerenreiner Ketone der Formel **VI** (wenn R⁶ und R⁷ ungleich sind) erhalten werden oder durch Racematspaltung über die Kristallisation diastereomerer Salze unter Verwendung chiraler Säuren, bevorzugt Weinsäure, Weinsäure-Derivate oder Mandelsäure (J. Gawroński, K. Gawrońska, Tartaric and Malic Acids in Synthesis, Wiley, New York 1999 und dort zitierte Literatur), hergestellt werden.

Die bei der Aminomethylierungsreaktion entstehenden diastereomeren Mannichbasen Iassen sich entweder durch säulenchromatographische Trennung oder durch fraktionierte Kristallisation ihrer Hydrochloride aus einem organischem Lösungsmittel wie z.B. 2-Butanon oder Aceton diastereomerenrein erhalten.

Ketone der Formel **VI** wurden entweder kommerziell erworben oder nach Literaturverfahren synthetisiert.

Ketone der Formel **VI** mit R⁷ C₁-C₆-Alkyl, Aryl, Heteroaryl, Alkylaryl oder Alkylheteroaryl lassen sich nach literaturbeschriebenen Verfahren durch α-Addition an Ketone der Formel **XII** erhalten.

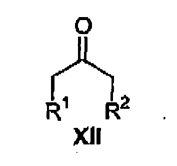

Für die regioselektive α-Addition über Enolether, Enolate, oder Enoxydialkylborane sind verschiedene Verfahren beschrieben worden (z.B. I. Kuwajima et al., J. Am. Chem. Soc. 1982, 104, 1025; H.O. House, Modern Synthetic Reactions, 2nd Ed., Benjamin, Menlo Park, California, 1972, J.K. Rasmussen, Synthesis 1977, 91, E.-i. Negishi et al, Tetrahedron Lett. 1979, 845). Die Verwendung von α-metallierten Hydrazonen von enolisierbaren Ketonen als Enolatequivalente über die Sequenz Carbonylverbindung, Hydrazon-Derivat, Metallierung, Umsetzung mit Halogeniden und anschließender Spaltung durch oxidativer Hydrolyse zur α-substituierten Carbonylverbindung ist von Enders et al. (D. Enders, W. Bettray, Pure Appl. Chem. 1996, 69, 569- 580. Recent Advances in the Development of Highly Enantioselective Synthetic Methods und D. Enders, in Asymmetric Synthesis Vol. 3, J.D. Morrison (Ed.), Academic Press, Orlando, 1984, 275 - 339, Alkylation of Chiral Hydrazones.) umfassend beschrieben worden. Nach diesen Verfahren sind auch durch Verwendung von chiralen Hydrazonen enantiomerenreine Ketone der Formel VI zugänglich (SAMP-Methode). Die Metallierung von Hydrazonen gelingt mit Basen wie z.B. Lithiumdiisopropylamid quantitativ, wobei die anschließende Umsetzung der metallierten Hydrazone mit Halogeniden zu den α-substituierten Hydrazonen ebenfalls in hohen Ausbeuten verläuft. Alernativ zur Umsetzung von metallierten Hydrazonen lassen sich auch Metalloenamine mit Halogeniden umsetzen (z.B. I. Paterson et al., Tetrahedron 1990, 46, 4663).

Ketone der allgemeinen Formel **VI** lassen sich auch durch Oxidation der entsprechenden Alkohole der allgemeinen Formel **XIII** darstellen, wobei R¹, R² und R⁷ die gleiche Bedeutung wie in der allgemeinen Formel **I** haben.

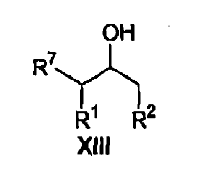

Verbindungen der Formel **XIII** lassen sich durch Hydroborierung, vorzugsweise unter Verwendung von 9-Borabicyclo-[3.3.0]nonan als Reduktionsmittel mit anschließender oxidativer Aufarbeitung vorzugsweise unter Verwendung von Wasserstoffperoxid im alkalischen pH-Wertbereich, aus den entsprechenden Olefinen der Formel **XIVa** und **XIVb** herstellen, wobei R¹, R² und R⁷ die gleiche Bedeutung wie in der allgemeinen Formel **I** haben (z.B. H.C. Brown et al., J. Am. Chem. Soc. 1977, 99, 3427).

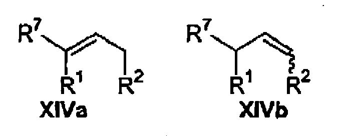

Hydroxyl-Derivate der Formel **XIII** können auch durch cuprat-katalysierte Öffnung der entsprechenden Oxiran-Derivate (Epoxide) der Formel **XV** durch Nukleophile, vorzugsweise Grignardverbindungen oder litiumorganische Verbindungen des Typs R⁷-Z **XVI**, erhalten werden (z.B. C. Huyn et al., Tetrahedron Lett. 1979, 1503; J.K.

Whitesell, et al., Chimia 1986, 40, 318), wobei Z die gleiche Bedeutung wie in Formel III hat und R⁷ C₁-C₆-Alkyl, Aryl, Heteroaryl, Alkylaryl oder Alkylheteroaryl ist. Durch enzymatische Racematspaltung lassen sich auch die enantiomerenreine Alkohole der **XIII** erhalten.

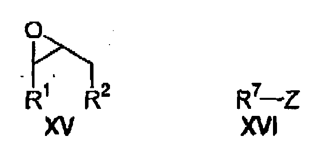

Alkohole der Formel **XIII**, in denen R⁷ ein Halogen darstellt, lassen sich z. B. durch Umsetzung der Oxiran-Derivate der Formel **XV** mit Litiumhalogeniden in Gegenwart von Säuren zu den entsprechenden Alkoholen der Formel **XIII** mit R⁷ Halogen umsetzen (J.S. Bagwa et al., Tetrahedron Lett. 1991, 32, 3021). Verbindungen der Formel (X), in denen R⁷ Brom ist, können durch Umsetzung der Olefine der Formel **(XIVa,b)** mit N-Bromsuccinimid in Wasser erhalten werden (C.O. Guss, J. Am. Chem. Soc. 1955, 77, 2549).

Substituierte 1-Aminobutan-3-ol-Derivate der Formel **I**, in denen R⁵ eine Phenolgruppe darstellt, lassen sich aus den Methoxyaryl-Derivaten durch selektive Etherspaltung z.B. mit Diisobutylaluminiumhydrid in einem aromatischen Kohlenwasserstoff, beispielsweise Toluol, bei einer Temperatur zwischen 60 und 130 °C herstellen (Synthesis 1975, 617; DBP 2409990, 2409991 und Chem. Abstr. 84, 59862 (19974)).

Weiterhin lassen sich 1-Aminobutan-3-ol-Derivate der Formel **I**, in denen R⁵ eine Phenolgruppe darstellt, aus den entsprechendem Benzyloxy-arylen durch reduktive Debenzylierung erhalten. Die Debenzylierung führt man bevorzugt in Gegenwart von Platin oder Palladium auf einem Trägermaterial wie Aktivkohle absorbiert als Katalysator in Gegenwart von Wasserstoff in einem Lösungsmittel wie Essigsäure oder einem C₁₋₄-Alkylalkohol bei Drücken von 1 bis 100 bar und Temperaturen von 20 - 100 °C durch.

### Salzbildung

Die Verbindungen der Formel **I** lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Acetylsalicylsäure, Hippursäure und/oder Asparaginsäure, in der sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon oder auch Wasser durchgeführt. Zur Herstellung der Hydrochloride eignet sich darüber hinaus Trimethylchlorsilan in wäßriger Lösung.

### Synthese der Verbindungen, bei denen R⁶ nicht Wasserstoff bedeutet:

Diese Verbindungen wurden nach Verfahren hergestellt, wie sie in der Literatur beschrieben sind, z. B.
Risch et al., Houben-Weyl - Methoden der Organischen Chemie, E21 b (1995) 1925-1929; Angew. Chem. 106 (1994) 2531-2533; Synlett (1997) 974-976.

Im folgenden wird die Erfindung weiter durch Beispiele erläutert, ohne sie darauf zu beschränken.

### Beispiele

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCl etc. oder synthetisiert).

Die Analytik erfolgte über ESI-Massenspektrometrie und/oder HPLC und/oder NMR-Spektroskopie.

In einem ausgeheizten und unter Inertgas auf - 10°C abgekühlten Reaktionsgefäß wurde die in THF gelöste Mannichbase (400µl, 0,5 mol/l) vorgelegt. Unter Rühren wurden daraufhin 2 Äquivalente des vorbereiteten Grignard- oder Organolithium-Reagenzes zugegeben (0,5 mol/l in THF oder Diethylether, 800 µl). Das Reaktionsgemisch wurde bei Raumtemperatur gerührt. Nach drei Stunden wurde erneut auf -10°C gekühlt und mit Ammoniumchlorid-Lösung hydrolisiert.
Das Reaktionsgemisch wurde zweimal mit Ethylacetat extrahiert und bei 40°C im Vakuum eingeengt.

Zur Charakterisierung der chlorierten Verbindungen wurde ein ESI-MS aufgenommen. Zur Charakterisierung der übrigen Verbindungen wurde ein NMR-Spektrum aufgenommen.

### Beispiel für die Synthese von

### 1-(3-Chlor-phenyl)-2-dimethylaminomethyl-6-(4-methoxy-phenyl)-cyclohexanol; Hydrochlorid

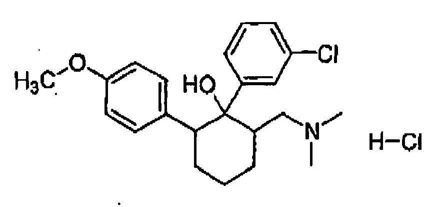

### 1. Stufe: 2-(4-Methoxy-phenyl)-cyclohexanol

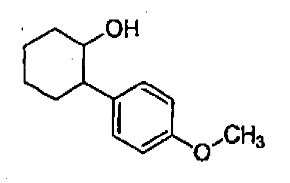

Die Reaktion wurde unter Stickstoff als Schutzgas durchgeführt. 19,4 g (0,8 mol) Magnesiumspäne wurden in 50 ml getrocknetem Tetrahydrofuran gerührt. Man tropfte 100 ml (150 g, 0,8 mol) 1-Brom-4-methoxy-benzol, gelöst in 300 ml absolutem Tertrahydrofuren, so zu, dass die Reaktionsmischung siedete. Anschließend wurde noch eine Stunde unter Rückfluß erhitzt bevor auf 10 °C abgekühlt wurde. Bei dieser Temperatur wurden portionsweise 14.9 g Kupferiodid zugegeben. Es wurde eine Stunde nachgerührt. Hiernach tropfte man 81 ml (78,6 g, 0,8 mol) 7-Oxa-bicyclo[4.1.0]heptan, gelöst in 160 ml getrocknetem Tetrahydrofuran so zu, dass die Innentemperatur der stark exothermen Reaktion 25 °C nicht überschritt (Aceton/Trockeneis-Kühlung). Nach beendeter Zugabe wurde über Nacht bei Raumtemperatur nachgerührt.
Zur Hydrolyse versetzte man das Reaktionsgemisch unter Eisbadkühlung zunächst mit 90 ml Wasser, bevor eine Mischung bestehend aus 17 g Ammoniumchlorid, 50 ml konzentrierter Salzsäure und 350 ml Wasser zugetropft wurde. Nach Phasentrennung wurde die wäßrige Phase dreimal mit 250 mi Diethylether extrahiert. Die vereinigten organischen Phasen wurden zweimal mit jeweils 150 ml gesättigter Natriumhydrogencarbonatlösung gewaschen. Nach Filtration über 200 g Kieselgel wurde über Natriumsulfat getrocknet. Nach destillativer Entfernung des Lösungsmittels wurden 150 g (89 % der Theorie) hellgelbe, wachsartige Kristalle erhalten, die ohne weitere Reinigung in die nächste Stufe eingesetzt wurden.

### 2. Stufe: 2-(4-Methoxy-phenyl)-cyclohexanon

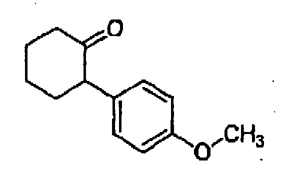

146 g (0,71 mol) 2-(4-Methoxy-phenyl)-cyclohexanol aus der 1. Stufe wurden in 600 ml Diethylether gelöst. Unter kräftigem Rühren wurde unter Eisbadkühlung eine Lösung bestehend aus 69,9 g (0,23 mol) Natriumdichromat (als Dihydrat), 355 ml Wasser und 53,2 ml konzentrierter Schwefelsäure so zugetropft, dass die Innentemperatur 10 °C nicht überschritt. Nach beendeter Zugabe wurde über Nacht bei Raumtemperatur nachgerührt. Nach Phasentrennung wurde die wäßrige Phase noch zweimal mit 200 ml Diethylether extrahiert. Die vereinigten organischen Phasen wurden zweimal mit 200 ml gesättigter Natriumhydrogencarbonatlösung gewaschen und über Natriumsulfat getrocknet. Nach destillativer Entfernung des Lösungsmittels wurde das zurückbleibende Öl destilliert und bei 128-134 °C/ 0,05 bar die übergehende Fraktion aufgefangen. Nach Kristallisation aus n-Hexan wurden 130 g farblose Kristalle mit einem Schmelzpunkt von 90 °C erhalten (90 % der Theorie).

### 3. Stufe: 2-Dimethylaminomethyl-6-(4-methoxy-phenyl)-cyclohexanon

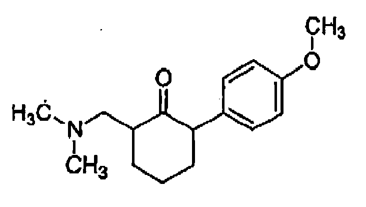

200 g (0,98 mol) 2-(4-Methoxy-phenyl)-cyclohexanon aus Stufe 2 und 91 g (1 mol) Dimethylammoniummethylenchlorid wurden in 1000 ml trockenem Acetonitril bei Raumtemperatur gerührt. Nach Zugabe von 1 ml Acetylchlorid wurde noch drei Stunden bei Raumtemperatur weitergerührt, wobei eine farblose, klare Lösung entstand. Anschließend wurden 2000 ml getrockneter Ether zum Reaktionsgemisch getropft, wobei das Hydrochlorid auskristallisierte. Es wurden 160 g (56 % der Theorie) farblose Kristalle erhalten. Aus dem Hydrochlorid wurde mit Dichlormethan/Natronlauge die Base freigesetzt und nach dem Trocknen der Lösung das Dichlormethan destillativ entfernt.

Dies ist ein allgemeingültiges Beispiel, an sich die Anwendung der literaturbekannten Verfahren zur Herstellung von Verbindungen mit R⁶ = H zeigen läßt.

### 4. Stufe: 1-(3-Chlor-phenyl)-2-dimethylaminomethyl-6-(4-methoxy-phenyl)-cyclohexanol; Hydrochlorid

Die Reaktion wurde unter Stickstoff als Schutzgas durchgeführt. 18.3 g (0.75 mol) Magnesiumspäne wurden in 150 ml getrocknetem Tetrahydrofuran gerührt. Man tropfte 88 ml (144 g, 0,75 mol) 1-Brom-3-chlor-benzol, gelöst in 500 ml absolutem Tertrahydrofuran, so zu, dass die Reaktionsmischung siedete. Anschließend wurde noch eine Stunde unter Rückfluß erhitzt bevor auf 10°C abgekühlt wurde. Hiernach tropfte 131 g (0,5 mol) 2-Dimethylaminomethyl-6-(4-methoxy-phenyl)-cyclohexanon aus Stufe 3, gelöst in 400 ml getrocknetem Tetrahydrofuran so zu, dass die Innentemperatur 20 °C nicht überschritt. Nach beendeter Zugabe wurde über Nacht bei Raumtemperatur nachgerührt.
Zur Hydrolyse versetzte man das Reaktionsgemisch unter Eisbadkühlung mit 1000 ml 20 %iger Ammoniumchlorid-Lösung. Nach Phasentrennung wurde die wäßrige Phase dreimal mit 250 ml Diethylether extrahiert. Die vereinigten organischen Phasen wurden zweimal mit jeweils 100 ml gesättigter Natriumhydrogencarbonatlösung gewaschen. Nach destillativer Entfernung des Lösungsmittels wurden 166 g (93 % der Theorie) hellgelbes Öl als Rohprodukt erhalten.
Die erhaltene Rohprodukt wurde auf eine 8 x 60 cm Säule, gefüllt mit Kieselgel, gegeben und mit Ethylacetat/Methanol im Verhältnis 1 : 1 eluiert.
Es wurden 91,7 g reine Base erhalten (52 % der Theorie). Die Base wurde in 920 ml 2-Butanon aufgenommen und mit 31,1 ml Trimethylchlorsilan und 4,4 ml Wasser versetzt. Über Nacht kristallisierten bei 4-5 °C 58,7 g (31,4 % der Theorie) Hydrochlorid mit einem Schmelzpunkt von 243 °C aus.

| **Liste der Beispiele:** | |
|---|---|
| | **Name** |
| Beispiel 1 | 2-Benzyl-1-(2,4-dichlor-benzyl)-6-dimethylaminomethyl-cyclohexanol |
| Beispiel 2 | 2-Benzyl-1-(3-chlor-benzyl)-6-dimethylaminomethyl-cyclohexanol |
| Beispiel 3 | 2-Benzyl-6-dimethylaminomethyl-1-(2,5-dimethyl-benzyl)-cyclohexanol |
| Beispiel 4 | 2-Benzyl-1-(2-chlor-6-fluor-benzyl)-6-dimethylaminomethyl-cyclohexanol |
| Beispiel 5 | 2-Benzyl-1-(4-chlor-benzyl)-6-dimethylaminomethyl-cyclohexanol |
| Beispiel 6 | 2-Benzyl-6-dimethylaminomethyl-1-(3-trifluormethyl-phenyl)-cyclohexanol |
| Beispiel 7 | 2-Benzyl-6-dimethylaminomethyl-1-(2-methyl-benzyl)-cyclohexanol |
| Beispiel 8 | 2-Benzyl-6-dimethylaminomethyl-1-(2-methoxy-phenyl)-cyclohexanol |
| Beispiel 9 | 2-Benzyl-1-(4-chlor-3-trifluormethyl-phenyl)-6-dimethylaminomethyl-cyclohexanol |
| Beispiel 10 | 2-Benzyl-1-(2-chlor-benzyl)-6-dimethylaminomethyl-cyclohexanol |
| Beispiel 11 | 2-Benzyl-1-(3,5-dichlor-phenyl)-6-dimethylaminomethyl-cyclohexanol |
| Beispiel 12 | 2-Benzyl-1-(3-chlor-phenyl)-6-dimethylaminomethyl-cyclohexanol |
| Beispiel 13 | 2-Benzyl-6-dimethylaminomethyl-1-(3-fluor-phenyl)-cyclohexanol |
| Beispiel 14 | 2-Benzyl-6-dimethylaminomethyl-1-(5-fluor-2-methoxy-phenyl)-cyclohexanol |
| Beispiel 15 | 2-Benzyl-1-cyclohexylmethyl-6-dimethylaminomethyl-cyclohexanol |
| Beispiel 16 | 2-Benzyl-6-dimethylaminomethyl-1-(4-methoxy-phenyl)-cyclohexanol |
| Beispiel 17 | 2-Benzyl-6-dimethylaminomethyl-1-p-tolyl-cyclohexanol |
| Beispiel 18 | 2-Benzyl-6-dimethylaminomethyl-1-(3-phenyl-propyl)-cyclohexanol |
| Beispiel 19 | 2-Benzyl-6-dimethylaminomethyl-1-(3-methoxy-phenyl)-cyclohexanol |
| Beispiel 20 | 2-Benzyl-6-dimethylaminomethyl-1-thiophen-2-yl-cyclohexanol |
| Beispiel 21 | 2-Benzyl-6-dimethylaminomethyl-1-phenylethynyl-cyclohexanol |
| Beispiel 22 | 2-Benzyl-6-dimethylaminomethyl-1-(4-fluor-phenyl)-cyclohexanol |
| Beispiel 23 | 2-Benzyl-6-dimethylaminomethyl-bicyclohexyl-1-ol |
| Beispiel 24 | 2-Benzyl-6-dimethylaminomethyl-1-m-tolyl-cyclohexanol |
| Beispiel 25 | 2-Benzyl-1-(4-tert-butyl-phenyl)-6-dimethylaminomethyl-cyclohexanol |
| Beispiel 26 | 2-Benzyl-6-dimethylaminomethyl-1-vinyl-cyclohexanol |
| Beispiel 27 | 2-Benzyl-6-dimethylaminomethyl-1-o-tolyl-cyclohexanol |
| Beispiel 28 | 2-Benzyl-6-dimethylaminomethyl-1-(4-fluor-3-methyl-phenyl)-cyclohexanol |
| Beispiel 29 | 1,2-Dibenzyl-6-dimethylaminomethyl-cyclohexanol |
| Beispiel 30 | 2-Benzyl-1-(4-chlor-phenyl)-6-dimethylaminomethyl-cyclohexanol |
| Beispiel 31 | 2-Benzyl-6-dimethylaminomethyl-1-phenyl-cyclohexanol |
| Beispiel 32 | 2-Dimethylaminomethyl-1-(2,5-dimethyl-phenyl)-6-(3-methoxy-phenyl)-cyclohexanol |
| Beispiel 33 | 1-Cyclohexylmethyl-2-dimethylaminomethyl-6-(3-methoxy-phenyl)-cyclohexanol |
| Beispiel 34 | 1-(2,4-Dichlor-benzyl)-2-dimethylaminomethyl-6-(3-methoxy-phenyl)-cyclohexanol |
| Beispiel 35 | 2-Dimethylaminomethyl-6-(3-methoxy-phenyl)-1-phenylethynyl-cyclohexanol |
| Beispiel 36 | 2-Dimethylaminomethyl-6-(3-methoxy-phenyl)-1-o-tolyl-cyclohexanol |
| Beispiel 37 | 1-(4-Chlor-phenyl)-2-dimethylaminomethyl-6-(3-methoxy-phenyl)-cyclohexanol |
| Beispiel 38 | 2-Dimethylaminomethyl-1-(3-fluor-phenyl)-6-(3-methoxy-phenyl)-cyclohexanol |
| Beispiel 39 | 2-Dimethylaminomethyl-1-(3-fluor-4-methoxy-phenyl)-6-(3-methoxy-phenyl)-cyclohexanol |
| Beispiel 40 | 1-(3-Chlor-benzyl)-2-dimethylaminomethyl-6-(3-methoxy-phenyl)-cyclohexanol |
| Beispiel 41 | 2-Dimethylaminomethyl-1-(3-methoxy-benzyl)-6-(3-methoxy-phenyl)-cyclohexanol |
| Beispiel 42 | 1-(4-Chlor-3-trifluormethyl-phenyl)-2-dimethylaminomethyl-6-(3-methoxy-phenyl)-cyclohexanol |
| Beispiel 43 | 1-(3,5-Dichlor-benzyl)-2-dimethylaminomethyl-6-(3-methoxy-phenyl)-cyclohexanol |
| Beispiel 44 | 2-Dimethylaminomethyl-6-(3-methoxy-phenyl)-1-phenylethynyl-cyclohexanol |
| Beispiel 45 | 1-(4-Chlor-phenyl)-2-dimethylaminomethyl-6-(3-methoxy-phenyl)-cyclohexanol |
| Beispiel 46 | 2-Dimethylaminomethyl-6-(3-methoxy-phenyl)-1-o-tolyl-cyclohexanol |
| Beispiel 47 | 1-(3-Chlor-benzyl)-2-dimethylaminomethyl-6-methyl-cyclohexanol |
| Beispiel 48 | 2-Dimethylaminomethyl-1-(2,5-dimethyl-benzyl)-6-methyl-cyclohexanol |
| Beispiel 49 | 1-(2-Chlor-6-fluor-benzyl)-2-dimethylaminomethyl-6-methyl-cyclohexanol |
| Beispiel 50 | 1-(4-Chlor-benzyl)-2-dimethylaminomethyl-6-methyl-cyclohexanol |
| Beispiel 51 | 2-Dimethylaminomethyl-6-methyl-1-(3-methyl-benzyl)-cyclohexanol |
| Beispiel 52 | 2-Dimethylaminomethyl-6-methyl-1-(3-trifluormethyl-phenyl)-cyclohexanol |
| Beispiel 53 | 1-(2-Chlor-3-fluor-phenyl)-2-dimethylaminomethyl-6-methyl-cyclohexanol |
| Beispiel 54 | 2-Dimethylaminomethyl-6-methyl-1-(2-methyl-benzyl)-cyclohexanol |
| Beispiel 55 | 2-Dimethylaminomethyl-1-(2-methoxy-phenyl)-6-methyl-cyclohexanol |
| Beispiel 56 | 2-Dimethylaminomethyl-1-(3-fluor-benzyl)-6-methyl-cyclohexanol |
| Beispiel 57 | 2-Dimethylaminomethyl-1-(4-fluor-benzyl)-6-methyl-cyclohexanol |
| Beispiel 58 | 1-(2-Chlor-benzyl)-2-dimethylaminomethyl-6-methyl-cyclohexanol |
| Beispiel 59 | 1-(3,5-Dichlor-phenyl)-2-dimethylaminomethyl-6-methyl-cyclohexanol |
| Beispiel 60 | 1-(3-Chlor-phenyl)-2-dimethylaminomethyl-6-methyl-cyclohexanol |
| Beispiel 61 | 2-Dimethylaminomethyl-1-(3-fluor-phenyl)-6-methyl-cyclohexanol |
| Beispiel 62 | 2-Dimethylaminomethyl-1-(3-fluor-4-methoxy-phenyl)-6-methyl-cyclohexanol |
| Beispiel 63 | 2-Dimethylaminomethyl-1-(4-methoxy-phenyl)-6-methyl-cyclohexanol |
| Beispiel 64 | 2-Dimethylaminomethyl-6-methyl-1-p-tolyl-cyclohexanol |
| Beispiel 65 | 2-Dimethylaminomethyl-6-methyl-1-(3-phenyl-propyl)-cyclohexanol |
| Beispiel 66 | 2-Dimethylaminomethyl-1-(3-methoxy-phenyl)-6-methyl-cyclohexanol |
| Beispiel 67 | 2-Dimethylaminomethyl-6-methyl-1-thiophen-2-yl-cyclohexanol |
| Beispiel 68 | 2-Dimethylaminomethyl-6-methyl-1-phenylethynyl-cyclohexanol |
| Beispiel 69 | 2-Dimethylaminomethyl-6-methyl-1-phenethyl-cyclohexanol |
| Beispiel 70 | 2-Dimethylaminomethyl-1-(4-fluor-phenyl)-6-methyl-cyclohexanol |
| Beispiel 71 | 1-(3-Chlor-benzyl)-2-dimethylaminomethyl-6-(3-methoxy-phenyl)-cyclohexanol |
| Beispiel 72 | 2-Dimethylaminomethyl-1-(4-fluor-2-methoxy-phenyl)-6-(3-methoxy-phenyl)-cyclohexanol |
| Beispiel 73 | 2-Dimethylaminomethyl-1-(2-methoxy-phenyl)-6-(3-methoxy-phenyl)-cyclohexanol |
| Beispiel 74 | 2-Dimethylaminomethyl-1-(4-fluor-benzyl)-6-(3-methoxy-phenyl)-cyclohexanol |
| Beispiel 75 | 1-(3-Chlor-phenyl)-2-dimethylaminomethyl-6-(3-methoxy-phenyl)-cyclohexanol |
| Beispiel 76 | 2-Dimethylaminomethyl-6-(3-methoxy-phenyl)-1-p-tolyl-cyclohexanol |
| Beispiel 77 | 2-Dimethylaminomethyl-6-(3-methoxy-phenyl)-1-(3-phenyl-propyl)-cyclohexanol |
| Beispiel 78 | 2-Dimethylaminomethyl-1,6-bis-(3-methoxy-phenyl)-cyclohexanol |
| Beispiel 79 | 2-Dimethylaminomethyl-6-(3-methoxy-phenyl)-1-thiophen-2-yl-cyclohexanol |
| Beispiel 80 | 2-Dimethylaminomethyl-6-(3-methoxy-phenyl)-1-phenethyl-cyclohexanol |
| Beispiel 81 | 3-[3-Dimethylaminomethyl-2-(4-fluor-phenyl)-2-hydroxy-cyclohexyl]-phenol |
| Beispiel 82 | 3-(3-Dimethylaminomethyl-2-hydroxy-2-phenyl-cyclohexyl)-phenol |
| Beispiel 83 | 3-[2-(4-tert-Butyl-phenyl)-3-dimethylaminomethyl-2-hydroxy-cyclohexyl]-phenol |
| Beispiel 84 | 3-(3-Dimethylaminomethyl-2-hydroxy-2-vinyl-cyclohexyl)-phenol |
| Beispiel 85 | 1-(4-Chlor-phenyl)-2-dimethylaminomethyl-6-(3-methoxy-phenyl)-cyclohexanol |
| Beispiel 86 | 3-Dimethylaminomethyl-2-(3-methoxy-phenyl)-bicyclohexyl-2-ol |
| Beispiel 87 | 2-Benzyl-6-dimethylaminomethyl-1-(4-trifluormethyl-phenyl)-cyclohexanol |
| Beispiel 88 | 3-(2-Benzyl-6-dimethylaminomethyl-1-hydroxy-cyclohexyl)-phenol |
| Beispiel 89 | 3-(2-*tert*-Butyl-6-dimethylaminomethyl-1-hydroxy-cyclohexyl)-phenol |

### Pharmakologische Untersuchungen

Beim Bindungsassay wird Gabapentin benutzt, um die Bindung und Affinitäten der ausgewählten Verbindungen zu überprüfen. Die Affinität der erfindungsgemäßen Verbindungen wird über die Verdrängung von Gabapentin von seiner Bindungsstelle gemessen. Wenn die ausgewählten Verbindungen Gabapentin von seiner Bindungsstelle verdrängen können, so kann man erwarten, daß sie dem Gabapentin vergleichbare pharmakologische Eigenschaften entfalten z.B. als Agenz gegen Schmerz oder Epilepsie. Die erfindungsgemäßen Verbindungen zeigen eine gute Hemmung (Verdrängung von Gabapentin) in diesem Assay. Die untersuchten Verbindungen weisen daher in diesem einem biochemischen Assay eine Affinität zur bislang unbekannten Gabapentin-Bindungsstelle auf.

| **Beispiel** | **%Hemmung Gabapentin, 10µMol** |
|---|---|
| 82 | 55 |
| 81 | 48 |
| 76 | 53 |
| 75 | 54 |
| 45 | 58 |

### Analgesieprüfung im Writhing-Test an der Maus

Die Untersuchung auf analgetische Wirksamkeit wurde im Phenylchinon-induzierten Writhing an der Maus (modifiziert nach I.C. Hendershot und J. Forsaith, J. Pharmacol. Exp. Ther. 1959, 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Äthanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Für einige Beispiele wurde die Zahl der reagierenden Tiere bestimmt:

| Beispiel | Reagierende Tiere/Kontrolltiere (Writhing) |
|---|---|
| 85 | 10/10 (46,4 mg/kg; iv) |
| 86 | 3/10 (21,5 mg/kg; po) |
| 87 | 2/10 (10 mg/kg; iv) |
| 88 | 2/10 (10 mg/kg; iv) |
| 89 | 3/10 (10 mg/kg; iv) |

## Patentansprüche

1. Substituierte 1-Aminobutan-3-ol-Derivate der allgemeinen Formel I, 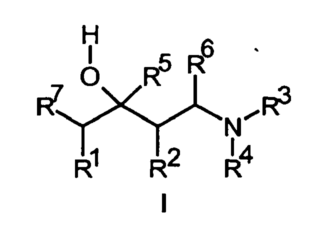 , worin
R¹ und R² zusammen einen (CH₂)₂₋₉-Ring bilden, der gegebenfalls mit C₁₋₈-Alkyl, verzweigt oder unverzweigt, gesättigt oder ungesättigt, unsubstituiert oder ein- oder mehrfach substituiert; oder Aryl, unsubstituiert oder ein- oder mehrfach substituiert; substituiert sein kann,
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; C₃₋₆-Cycloalkyl, gesättigt oder ungesättigt, unsubstituiert oder ein- oder mehrfach substituiert; Phenyl, Benzyl oder Phenethyl, unsubstituiert oder ein- oder mehrfach substituiert oder
die Reste R³ und R⁴ zusammen einen Ring bilden und
CH₂CH₂OCH₂CH₂, CH₂CH₂NR²²CH₂CH₂ oder (CH₂)₃₋₆ bedeuten,
mit R²² ausgewählt aus H; C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl, gesättigt oder ungesättigt, gebundenem Aryl, C₃₋₁₀-Cycloalkyl oder Heteroaryl, jeweils einfach oder mehrfach substituiert oder unsubstituiert;
R⁵ ausgewählt ist aus C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt; Aryl, Heteroaryl, über gesättigtes oder ungesättigtes C₁₋₃-Alkyl gebundenem Aryl, über gesättigtes oder ungesättigtes C₁₋₃-Alkyl gebundenem C₃₋₁₀-Cycloalkyl oder über gesättigtes oder ungesättigtes C₁₋₃-Alkyl gebundenem Heteroaryl, wobei alle Aryl, Heteroaryl und Cycloalkyl-Reste jeweils unabhängig voneinander unsubstituiert oder einfach oder mehrfach substituiert sein können mit Resten unabhängig voneinander ausgewählt aus
F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
mit R¹⁸ ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
R¹⁹ und R²⁰ unabhängig voneinander ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
oder R¹⁹ und R²⁰ zusammen CH₂CH₂OCH₂CH₂, CH₂CH₂NR²¹CH₂CH₂ oder (CH₂)₃₋₆ bilden,
mit R²¹ ausgewählt aus H, C₁₋₁₀-Alkyl gesättigt oder ungesättigt, verzweigt oder unverzweigt, unsubstituiert oder einfach oder mehrfach substituiert;
R⁶ ausgewählt ist aus H; Aryl oder Heteroaryl; jeweils unsubstituiert oder ein- oder mehrfach substituiert; und
R⁷ ausgewählt ist aus Halogen, CF_{3;} C₃-C₉-Cycloalkyl, gesättigt oder ungesättigt, unsubstituiert oder ein- oder mehrfach substituiert; Aryl, Heteroaryl, jeweils unsubstituiert oder ein- oder mehrfach substituiert; über gesättigtes oder ungesättigtes C₁₋₃-Alkyl gebundenem Aryl, C₃₋₉-Cycloalkyl oder Heteroaryl, jeweils unsubstituiert oder ein- oder mehrfach substituiert
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

2. Substituierte 1-Aminobutan-3-ol-Derivate gemäß Anspruch 1, **dadurch gekennzeichnet, daß**
R6 ausgewählt ist aus H oder Heteroaryl oder vorzugsweise ein Rest gemäß Formel II ist, 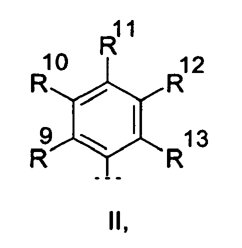 mit R⁹ bis R¹³, jeweils unabhängig voneinander ausgewählt aus H, F, Cl, Br, I, CF₃, OH, OR¹⁴, OCF₃, Sr¹⁴, SO₂CH₃, SO₂CF₃; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert; CN, COOR¹⁴, NO₂ oder
R⁹ und R¹⁰ oder R¹⁰ und R¹¹ zusammen einen OCH₂O- oder OCH₂CH₂O-Ring bilden, und
R¹⁴ ausgewählt ist aus C₁₋₆-Alkyl, verzweigt oder unverzweigt, gesättigt oder ungesättigt, unsubstituiert oder ein- oder mehrfach substituiert; Phenyl, Benzyl, Phenethyl oder Thiophen, jeweils unsubstituiert oder ein- oder mehrfach substituiert.

3. Substituierte 1-Aminobutan-3-ol-Derivate gemäß einem der Ansprüche 1 oder 2, **dadurch gekennzeichnet, daß**
R¹ und R² zusammen einen (CH₂)₂₋₅-Ring bilden, der gegebenfalls mit C₁₋₆-Alkyl, verzweigt oder unverzweigt, gesättigt oder ungesättigt, unsubstituiert oder ein- oder mehrfach substituiert; oder Phenyl, unsubstituiert oder ein- oder mehrfach substituiert; substituiert sein kann, der aber vorzugsweise unsubstituiert ist.

4. Substituierte 1-Aminobutan-3-ol-Derivate gemäß einem der Ansprüche 1 bis 3, **dadurch gekennzeichnet, daß**
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; vorzugsweise beide CH₃ sind,
oder die Reste R³ und R⁴ zusammen einen Ring bilden und CH₂CH₂NR²²CH₂CH₂ oder (CH₂)₃₋₆ bedeuten, insbesondere zusammen oder (CH₂)₄₋₅ oder CH₂CH₂NR²²CH₂CH₂ bedeuten, mit R²² ausgewählt aus H oder C₁₋₆-Alkyl, gesättigt, verzweigt oder unverzweigt und unsubstituiert; insbesondere H oder CH₃;

5. Substituierte 1-Aminobutan-3-ol-Derivate gemäß einem der Ansprüche 1 bis 4, **dadurch gekennzeichnet, daß**
R⁵ ausgewählt ist aus C₁₋₆-Alkyl, gesättigt oder ungesättigt, verzweigt oder unverzweigt, einfach oder mehrfach substituiert oder unsubstituiert; C₅₋₆-Cycloalkyl, Phenyl, Thiophenyl, Furyl, Benzofuranyl, Benzothiophenyl, Pyrrolyl, Pyridinyl, Pyrimidinyl, Chinolinyl, Isochinolinyl, Chinazolinyl, über gesättigtes oder ungesättigtes C₁₋₃-Alkyl gebundenem Phenyl, über gesättigtes oder ungesättigtes C₁₋₃-Alkyl gebundenem C₅₋₆-Cycloalkyl oder über gesättigtes oder ungesättigtes C₁₋₃-Alkyl gebundenem Thiophenyl, Furyl, Benzofuranyl, Benzothiophenyl, Pyrrolyl, Pyridinyl, Pyrimidinyl, Chinolinyl, Isochinolinyl, Chinazolinyl, wobei alle Aryl, Heteroaryl und Cycloalkyl-Reste jeweils unabhängig voneinander unsubstituiert oder einfach oder mehrfach substituiert sein können,
vorzugsweise
R⁵ ausgewählt ist aus Phenyl oder Thiophenyl, unsubstituiert oder einfach oder mehrfach, vorzugsweise mit F, Cl, Br, I, OH, O-C₁₋₄-Alkyl, C₁₋₆-Alkyl, NH₂ und/oder SH, substituiert; oder über gesättigtes oder ungesättigtes C₁₋₃-Alkyl gebundenem Phenyl, unsubstituiert oder einfach oder mehrfach, vorzugsweise mit F, Cl, Br, I, OH, O-C₁₋₄-Alkyl, C₁₋₆-Alkyl, NH₂ und/oder SH, substituiert,
insbesondere
R⁵ ausgewählt ist aus Phenyl unsubstituiert oder einfach oder mehrfach, vorzugsweise mit F, Cl, Br, I, OH, OCH₃, OC₂H₅, OC₃H₇, SH, CH₃, C₂H₅, C₃H₇ und/oder C₄H₉, substituiert.

6. Substituierte 1-Aminobutan-3-ol-Derivate gemäß einem der Ansprüche 1 bis 5, **dadurch gekennzeichnet, daß**
R⁷ ausgewählt ist aus C₅-C₇-Cycloalkyl, gesättigt oder ungesättigt, unsubstituiert oder ein- oder mehrfach substituiert, vorzugsweise Cyclohexyl; Phenyl, unsubstituiert oder ein- oder mehrfach substituiert; über gesättigtes oder ungesättigtes C₁₋₃-Alkyl gebundenem Phenyl, jeweils unsubstituiert oder ein- oder mehrfach substituiert;
vorzugsweise R⁷ ausgewählt ist aus Phenyl, unsubstituiert oder ein- oder mehrfach substituiert; über gesättigtes oder ungesättigtes C₁₋₃-Alkyl gebundenem Phenyl, jeweils unsubstituiert oder ein- oder mehrfach substituiert.

7. Substituierte 1-Aminobutan-3-ol-Derivate gemäß einem der Ansprüche 1 bis 6, **dadurch gekennzeichnet, daß**, wenn R5 und/oder R7 ausgewählt ist aus über gesättigtes oder ungesättigtes C₁₋₃-Alkyl gebundenem Aryl, C₃₋₉-Cycloalkyl oder Heteroaryl, das C₁₋₃-Alkyl über das Aryl, Heteroaryl oder Cycloalkyl gebunden ist, ausgewählt ist aus:
-CH₂-, -C₂H₄-, C₃H₆-, -C≡C-, -CH=CH-, -CH=CH-CH₂-, -CH₂-CH=CH-, - C≡C-CH₂- oder -CH2-C≡C-, vorzugsweise -CH₂-, -C₂H₄- oder -C≡C-.

8. Substituierte 1-Aminobutan-3-ol-Derivate gemäß einem der Ansprüche 1 bis 7, **dadurch gekennzeichnet, daß** sie ausgewählt sind aus der folgenden Gruppe:
• 2-Benzyl-1-(2,4-dichlor-benzyl)-6-dimethylaminomethyl-cyclohexanol
• 2-Benzyl-1-(3-chlor-benzyl)-6-dimethylaminomethyl-cyclohexanol
• 2-Benzyl-6-dimethylaminomethyl-1-(2,5-dimethyl-benzyl)-cyclohexanol
• 2-Benzyl-1-(2-chlor-6-fluor-benzyl)-6-dimethylaminomethyl-cyclohexanol
• 2-Benzyl-1-(4-chlor-benzyl)-6-dimethylaminomethyl-cyclohexanol
• 2-Benzyl-6-dimethylaminomethyl-1-(3-trifluormethyl-phenyl)-cyclohexanol
• 2-Benzyl-6-dimethylaminomethyl-1-(2-methyl-benzyl)-cyclohexanol
• 2-Benzyl-6-dimethylaminomethyl-1-(2-methoxy-phenyl)-cyclohexanol
• 2-Benzyl-1-(4-chlor-3-trifluormethyl-phenyl)-6-dimethylaminomethylcyclohexanol
• 2-Benzyl-1-(2-chlor-benzyl)-6-dimethylaminomethyl-cyclohexanol
• 2-Benzyl-1-(3,5-dichlor-phenyl)-6-dimethylaminomethyl-cyclohexanol
• 2-Benzyl-1-(3-chlor-phenyl)-6-dimethylaminomethyl-cyclohexanol
• 2-Benzyl-6-dimethylaminomethyl-1-(3-fluor-phenyl)-cyclohexanol
• 2-Benzyl-6-dimethylaminomethyl-1-(5-fluor-2-methoxy-phenyl)-cyclohexanol
• 2-Benzyl-1-cyclohexylmethyl-6-dimethylaminomethyl-cyclohexanol
• 2-Benzyl-6-dimethylaminomethyl-1-(4-methoxy-phenyl)-cyclohexanol
• 2-Benzyl-6-dimethylaminomethyl-1-p-tolyl-cyclohexanol
• 2-Benzyl-6-dimethylaminomethyl-1-(3-phenyl-propyl)-cyclohexanol
• 2-Benzyl-6-dimethylaminomethyl-1-(3-methoxy-phenyl)-cyclohexanol
• 2-Benzyl-6-dimethylaminomethyl-1-thiophen-2-yl-cyclohexanol
• 2-Benzyl-6-dimethylaminomethyl-1-phenylethynyl-cyclohexanol
• 2-Benzyl-6-dimethylaminomethyl-1-(4-fluor-phenyl)-cyclohexanol
• 2-Benzyl-6-dimethylaminomethyl-bicyclohexyl-1-ol
• 2-Benzyl-6-dimethylaminomethyl-1-m-tolyl-cyclohexanol
• 2-Benzyl-1-(4-tert-butyl-phenyl)-6-dimethylaminomethyl-cyclohexanol
• 2-Benzyl-6-dimethylaminomethyl-1-vinyl-cyclohexanol
• 2-Benzyl-6-dimethylaminomethyl-1-o-tolyl-cyclohexanol
• 2-Benzyl-6-dimethylaminomethyl-1-(4-fluor-3-methyl-phenyl)-cyclohexanol
• 1,2-Dibenzyl-6-dimethylaminomethyl-cyclohexanol
• 2-Benzyl-1-(4-chlor-phenyl)-6-dimethylaminomethyl-cyclohexanol
• 2-Benzyl-6-dimethylaminomethyl-1-phenyl-cyclohexanol
• 2-Dimethylaminomethyl-1-(2,5-dimethyl-phenyl)-6-(3-methoxy-phenyl)-cyclohexanol
• 1-Cyclohexylmethyl-2-dimethylaminomethyl-6-(3-methoxy-phenyl)-cyclohexanol
• 1-(2,4-Dichlor-benzyl)-2-dimethylaminomethyl-6-(3-methoxy-phenyl)-cyclohexanol
• 2-Dimethylaminomethyl-6-(3-methoxy-phenyl)-1-phenylethynylcyclohexanol
• 2-Dimethylaminomethyl-6-(3-methoxy-phenyl)-1-o-tolyl-cyclohexanol
• 1-(4-Chlor-phenyl)-2-dimethylaminomethyl-6-(3-methoxy-phenyl)-cyclohexanol
• 2-Dimethylaminomethyl-1-(3-fluor-phenyl)-6-(3-methoxy-phenyl)-cyclohexanol
• 2-Dimethylaminomethyl-1-(3-fluor-4-methoxy-phenyl)-6-(3-methoxy-phenyl)-cyclohexanol
• 1-(3-Chlor-benzyl)-2-dimethylaminomethyl-6-(3-methoxy-phenyl)-cyclohexanol
• 2-Dimethylaminomethyl-1-(3-methoxy-benzyl)-6-(3-methoxy-phenyl)-cyclohexanol
• 1-(4-Chlor-3-trifluormethyl-phenyl)-2-dimethylaminomethyl-6-(3-methoxyphenyl)-cyclohexanol
• 1-(3,5-Dichlor-benzyl)-2-dimethylaminomethyl-6-(3-methoxy-phenyl)-cyclohexanol
• 2-Dimethylaminomethyl-6-(3-methoxy-phenyl)-1-phenylethynylcyclohexanol
• 1-(4-Chlor-phenyl)-2-dimethylaminomethyl-6-(3-methoxy-phenyl)-cyclohexanol
• 2-Dimethylaminomethyl-6-(3-methoxy-phenyl)-1-o-tolyl-cyclohexanol
• 1-(3-Chlor-benzyl)-2-dimethylaminomethyl-6-(3-methoxy-phenyl)-cyclohexanol
• 2-Dimethylaminomethyl-1-(4-fluor-2-methoxy-phenyl)-6-(3-methoxy-phenyl)-cyclohexanol
• 2-Dimethylaminomethyl-1-(2-methoxy-phenyl)-6-(3-methoxy-phenyl)-cyclohexanol
• 2-Dimethylaminomethyl-1-(4-fluor-benzyl)-6-(3-methoxy-phenyl)-cyclohexanol
• 1-(3-Chlor-phenyl)-2-dimethylaminomethyl-6-(3-methoxy-phenyl)-cyclohexanol
• 2-Dimethylaminomethyl-6-(3-methoxy-phenyl)-1-p-tolyl-cyclohexanol
• 2-Dimethylaminomethyl-6-(3-methoxy-phenyl)-1-(3-phenyl-propyl)-cyclohexanol
• 2-Dimethylaminomethyl-1,6-bis-(3-methoxy-phenyl)-cyclohexanol
• 2-Dimethylaminomethyl-6-(3-methoxy-phenyl)-1-thiophen-2-yl-cyclohexanol
• 2-Dimethylaminomethyl-6-(3-methoxy-phenyl)-1-phenethyl-cyclohexanol
• 3-[3-Dimethylaminomethyl-2-(4-fluor-phenyl)-2-hydroxy-cyclohexyl]-phenol
• 3-(3-Dimethylaminomethyl-2-hydroxy-2-phenyl-cyclohexyl)-phenol
• 3-[2-(4-tert-Butyl-phenyl)-3-dimethylaminomethyl-2-hydroxy-cyclohexyl]-phenol
• 3-(3-Dimethylaminomethyl-2-hydroxy-2-vinyl-cyclohexyl)-phenol
• 1-(4-Chlor-phenyl)-2-dimethylaminomethyl-6-(3-methoxy-phenyl)-cyclohexanol
• 3-Dimethylaminomethyl-2-(3-methoxy-phenyl)-bicyclohexyl-2-ol
• 2-Benzyl-6-dimethylaminomethyl-1-(4-trifluormethyl-phenyl)-cyclohexanol
• 3-(2-Benzyl-6-dimethylaminomethyl-1-hydroxy-cyclohexyl)-phenol
• 3-(2-tert-Butyl-6-dimethylaminomethyl-1-hydroxy-cyclohexyl)-phenol
in Form ihrer reinen Stereoisomeren, insbesondere Enantiomeren oder Diastereomeren.

9. Arzneimittel enthaltend wenigstens ein substituiertes 1-Aminobutan-3-ol-Derivat der allgemeinen Formel I, 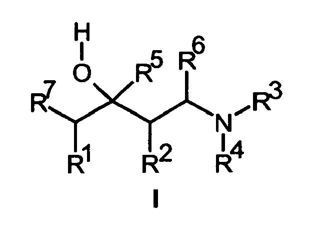 , worin
R¹ und R² zusammen einen (CH₂)₂₋₉-Ring bilden, der gegebenfalls mit C₁₋₈-Alkyl, verzweigt oder unverzweigt, gesättigt oder ungesättigt, unsubstituiert oder ein- oder mehrfach substituiert; oder Aryl, unsubstituiert oder ein- oder mehrfach substituiert; substituiert sein kann,
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; C₃₋₆-Cycloalkyl, gesättigt oder ungesättigt, unsubstituiert oder ein- oder mehrfach substituiert; Phenyl, Benzyl oder Phenethyl, unsubstituiert oder ein- oder mehrfach substituiert oder
die Reste R³ und R⁴ zusammen einen Ring bilden und CH₂CH₂OCH₂CH₂, CH₂CH₂NR²²CH₂CH₂ oder (CH₂)₃₋₆ bedeuten,
mit R²² ausgewählt aus H; C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl, gesättigt oder ungesättigt, gebundenem Aryl, C₃₋₁₀-Cycloalkyl oder Heteroaryl, jeweils einfach oder mehrfach substituiert oder unsubstituiert;
R⁵ ausgewählt ist aus C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt; Aryl, Heteroaryl, über gesättigtes oder ungesättigtes C₁₋₃-Alkyl gebundenem Aryl, über gesättigtes oder ungesättigtes C₁₋₃-Alkyl gebundenem C₃₋₁₀-Cycloalkyl oder über gesättigtes oder ungesättigtes C₁₋₃-Alkyl gebundenem Heteroaryl, wobei alle Aryl, Heteroaryl und Cycloalkyl-Reste jeweils unabhängig voneinander unsubstituiert oder einfach oder mehrfach substituiert sein können mit Resten unabhängig voneinander ausgewählt aus
F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
mit R¹⁸ ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
R¹⁹ und R²⁰ unabhängig voneinander ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
oder R¹⁹ und R²⁰ zusammen CH₂CH₂OCH₂CH₂, CH₂CH₂NR²¹CH₂CH₂ oder (CH₂)₃₋₆ bilden,
mit R²¹ ausgewählt aus H, C₁₋₁₀-Alkyl gesättigt oder ungesättigt, verzweigt oder unverzweigt, unsubstituiert oder einfach oder mehrfach substituiert;
R⁶ ausgewählt ist aus H; Aryl oder Heteroaryl; jeweils unsubstituiert oder ein- oder mehrfach substituiert; und
R⁷ ausgewählt ist aus Halogen, CF_{3;} C₁-C₆-Alkyl, verzweigt oder unverzweigt, gesättigt oder ungesättigt, unsubstituiert oder ein- oder mehrfach substituiert; C₃-C₉-Cycloalkyl, gesättigt oder ungesättigt, unsubstituiert oder ein- oder mehrfach substituiert; Aryl, Heteroaryl, jeweils unsubstituiert oder ein- oder mehrfach substituiert; über gesättigtes oder ungesättigtes C₁₋₃-Alkyl gebundenem Aryl, C₃₋₉-Cycloalkyl oder Heteroaryl, jeweils unsubstituiert oder ein- oder mehrfach substituiert;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate,
sowie gegebenenfalls geeignete Zusatz- und/oder Hilfsstoffe und/oder gegebenenfalls weitere Wirkstoffe.

10. Arzneimittel gemäß Anspruch 9, **dadurch gekennzeichnet, daß**
R6 ausgewählt ist aus H oder Heteroaryl oder vorzugsweise ein Rest gemäß Formel II ist, 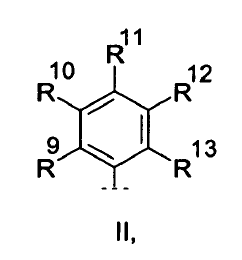 mit R⁹ bis R¹³, jeweils unabhängig voneinander ausgewählt aus H, F, Cl, Br, I, CF₃, OH, OR¹⁴, OCF₃, SR¹⁴, SO₂CH₃, SO₂CF_{3;} C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert; CN, COOR¹⁴, NO₂ oder
R⁹ und R¹⁰ oder R¹⁰ und R¹¹ zusammen einen OCH₂O- oder OCH₂CH₂O-Ring bilden, und
R¹⁴ ausgewählt ist aus C₁₋₆-Alkyl, verzweigt oder unverzweigt, gesättigt oder ungesättigt, unsubstituiert oder ein- oder mehrfach substituiert; Phenyl, Benzyl, Phenethyl oder Thiophen, jeweils unsubstituiert oder ein- oder mehrfach substituiert.

11. Arzneimittel gemäß Anspruch 9 oder 10, **dadurch gekennzeichnet, daß**
R¹ und R² zusammen einen (CH₂)₂₋₅-Ring bilden, der gegebenfalls mit C₁₋₆-Alkyl, verzweigt oder unverzweigt, gesättigt oder ungesättigt, unsubstituiert oder ein- oder mehrfach substituiert; oder Phenyl, unsubstituiert oder ein- oder mehrfach substituiert; substituiert sein kann, der aber vorzugsweise unsubstituiert ist.

12. Arzneimittel gemäß einem oder mehreren der Ansprüche 9 bis 11, **dadurch gekennzeichnet, daß**
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; vorzugsweise beide CH₃ sind,
oder die Reste R³ und R⁴ zusammen einen Ring bilden und CH₂CH₂NR²²CH₂CH₂ oder (CH₂)₃₋₆ bedeuten, insbesondere zusammen oder (CH₂)₄₋₅ oder CH₂CH₂NR²²CH₂CH₂ bedeuten, mit R²² ausgewählt aus H oder C₁₋₆-Alkyl, gesättigt, verzweigt oder unverzweigt und unsubstituiert; insbesondere H oder CH₃;

13. Arzneimittel gemäß einem oder mehreren der Ansprüche 9 bis 12, **dadurch gekennzeichnet, daß**
R⁵ ausgewählt ist aus C₁₋₆-Alkyl, gesättigt oder ungesättigt, verzweigt oder unverzweigt, einfach oder mehrfach substituiert oder unsubstituiert; C₅₋₆-Cycloalkyl, Phenyl, Thiophenyl, Furyl, Benzofuranyl, Benzothiophenyl, Pyrrolyl, Pyridinyl, Pyrimidinyl, Chinolinyl, Isochinolinyl, Chinazolinyl, über gesättigtes oder ungesättigtes C₁₋₃-Alkyl gebundenem Phenyl, über gesättigtes oder ungesättigtes C₁₋₃-Alkyl gebundenem C₅₋₆-Cycloalkyl oder über gesättigtes oder ungesättigtes C₁₋₃-Alkyl gebundenem Thiophenyl, Furyl, Benzofuranyl, Benzothiophenyl, Pyrrolyl, Pyridinyl, Pyrimidinyl, Chinolinyl, Isochinolinyl, Chinazolinyl, wobei alle Aryl, Heteroaryl und Cycloalkyl-Reste jeweils unabhängig voneinander unsubstituiert oder einfach oder mehrfach substituiert sein können,
vorzugsweise
R⁵ ausgewählt ist aus Phenyl oder Thiophenyl, unsubstituiert oder einfach oder mehrfach, vorzugsweise mit F, Cl, Br, I, OH, O-C₁₋₄-Alkyl, C₁₋₆-Alkyl, NH₂ und/oder SH, substituiert; oder über gesättigtes oder ungesättigtes C₁₋₃-Alkyl gebundenem Phenyl, unsubstituiert oder einfach oder mehrfach, vorzugsweise mit F, Cl, Br, I, OH, O-C₁₋₄-Alkyl, C₁₋₆-Alkyl, NH₂ und/oder SH, substituiert,
insbesondere
R⁵ ausgewählt ist aus Phenyl unsubstituiert oder einfach oder mehrfach, vorzugsweise mit F, Cl, Br, I, OH, OCH₃, OC₂H₅, OC₃H₇, SH, CH₃, C₂H₅, C₃H₇ und/oder C₄H₉, substituiert.

14. Arzneimittel gemäß einem oder mehreren der Ansprüche 9 bis 13, **dadurch gekennzeichnet, daß**
R⁷ ausgewählt ist aus C₁-C₆-Alkyl, verzweigt oder unverzweigt, gesättigt oder ungesättigt, unsubstituiert oder ein- oder mehrfach substituiert; C₅-C₇-Cycloalkyl, gesättigt oder ungesättigt, unsubstituiert oder ein- oder mehrfach substituiert, vorzugsweise Cyclohexyl; Phenyl, unsubstituiert oder ein- oder mehrfach substituiert; über gesättigtes oder ungesättigtes C₁₋₃-Alkyl gebundenem Phenyl, jeweils unsubstituiert oder ein- oder mehrfach substituiert;
vorzugsweise R⁷ ausgewählt ist aus Phenyl, unsubstituiert oder ein- oder mehrfach substituiert; über gesättigtes oder ungesättigtes C₁₋₃-Alkyl gebundenem Phenyl, jeweils unsubstituiert oder ein- oder mehrfach substituiert.

15. Arzneimittel gemäß einem oder mehreren der Ansprüche 9 bis 14, **dadurch gekennzeichnet, daß**, wenn R5 und/oder R7 ausgewählt ist aus über gesättigtes oder ungesättigtes C₁₋₃-Alkyl gebundenem Aryl, C₃₋₉-Cycloalkyl oder Heteroaryl, das C₁₋₃-Alkyl über das Aryl, Heteroaryl oder Cycloalkyl gebunden ist, ausgewählt ist aus:
- CH₂-, -C₂H₄-, C₃H₆-, -C≡C-, -CH=CH-, -CH=CH-CH₂-, -CH₂-CH=CH-, - C≡C-CH₂- oder -CH2-C≡C-, vorzugsweise -CH₂-, -C₂H₄- oder -C≡C-.

16. Arzneimittel gemäß einem oder mehreren der Ansprüche 9-15, **dadurch gekennzeichnet, daß** die substituierten 1-Aminobutan-3-ol-Derivatn ausgewählt sind aus der folgenden Gruppe:
• 2-Benzyl-1-(2,4-dichlor-benzyl)-6-dimethylaminomethyl-cyclohexanol
• 2-Benzyl-1-(3-chlor-benzyl)-6-dimethylaminomethyl-cyclohexanol
• 2-Benzyl-6-dimethylaminomethyl-1-(2,5-dimethyl-benzyl)-cyclohexanol
• 2-Benzyl-1-(2-chlor-6-fluor-benzyl)-6-dimethylaminomethyl-cyclohexanol
• 2-Benzyl-1-(4-chlor-benzyl)-6-dimethylaminomethyl-cyclohexanol
• 2-Benzyl-6-dimethylaminomethyl-1-(3-trifluormethyl-phenyl)-cyclohexanol
• 2-Benzyl-6-dimethylaminomethyl-1-(2-methyl-benzyl)-cyclohexanol
• 2-Benzyl-6-dimethylaminomethyl-1-(2-methoxy-phenyl)-cyclohexanol
• 2-Benzyl-1-(4-chlor-3-trifluormethyl-phenyl)-6-dimethylaminomethylcyclohexanol
• 2-Benzyl-1-(2-chlor-benzyl)-6-dimethylaminomethyl-cyclohexanol
• 2-Benzyl-1-(3,5-dichlor-phenyl)-6-dimethylaminomethyl-cyclohexanol
• 2-Benzyl-1-(3-chlor-phenyl)-6-dimethylaminomethyl-cyclohexanol
• 2-Benzyl-6-dimethylaminomethyl-1-(3-fluor-phenyl)-cyclohexanol
• 2-Benzyl-6-dimethylaminomethyl-1-(5-fluor-2-methoxy-phenyl)-cyclohexanol
• 2-Benzyl-1-cyclohexylmethyl-6-dimethylaminomethyl-cyclohexanol
• 2-Benzyl-6-dimethylaminomethyl-1-(4-methoxy-phenyl)-cyclohexanol
• 2-Benzyl-6-dimethylaminomethyl-1-p-tolyl-cyclohexanol
• 2-Benzyl-6-dimethylaminomethyl-1-(3-phenyl-propyl)-cyclohexanol
• 2-Benzyl-6-dimethylaminomethyl-1-(3-methoxy-phenyl)-cyclohexanol
• 2-Benzyl-6-dimethylaminomethyl-1-thiophen-2-yl-cyclohexanol
• 2-Benzyl-6-dimethylaminomethyl-1-phenylethynyl-cyclohexanol
• 2-Benzyl-6-dimethylaminomethyl-1-(4-fluor-phenyl)-cyclohexanol
• 2-Benzyl-6-dimethylaminomethyl-bicyclohexyl-1-ol
• 2-Benzyl-6-dimethylaminomethyl-1-m-tolyl-cyclohexanol
• 2-Benzyl-1-(4-tert-butyl-phenyl)-6-dimethylaminomethyl-cyclohexanol
• 2-Benzyl-6-dimethylaminomethyl-1-vinyl-cyclohexanol
• 2-Benzyl-6-dimethylaminomethyl-1-o-tolyl-cyclohexanol
• 2-Benzyl-6-dimethylaminomethyl-1-(4-fluor-3-methyl-phenyl)-cyclohexanol
• 1,2-Dibenzyl-6-dimethylaminomethyl-cyclohexanol
• 2-Benzyl-1-(4-chlor-phenyl)-6-dimethylaminomethyl-cyclohexanol
• 2-Benzyl-6-dimethylaminomethyl-1-phenyl-cyclohexanol
• 2-Dimethylaminomethyl-1-(2,5-dimethyl-phenyl)-6-(3-methoxy-phenyl)-cyclohexanol
• 1-Cyclohexylmethyl-2-dimethylaminomethyl-6-(3-methoxy-phenyl)-cyclohexanol
• 1-(2,4-Dichlor-benzyl)-2-dimethylaminomethyl-6-(3-methoxy-phenyl)-cyclohexanol
• 2-Dimethylaminomethyl-6-(3-methoxy-phenyl)-1-phenylethynylcyclohexanol
• 2-Dimethylaminomethyl-6-(3-methoxy-phenyl)-1-o-tolyl-cyclohexanol
• 1-(4-Chlor-phenyl)-2-dimethylaminomethyl-6-(3-methoxy-phenyl)-cyclohexanol
• 2-Dimethylaminomethyl-1-(3-fluor-phenyl)-6-(3-methoxy-phenyl)-cyclohexanol
• 2-Dimethylaminomethyl-1-(3-fluor-4-methoxy-phenyl)-6-(3-methoxy-phenyl)-cyclohexanol
• 1-(3-Chlor-benzyl)-2-dimethylaminomethyl-6-(3-methoxy-phenyl)-cyclohexanol
• 2-Dimethylaminomethyl-1-(3-methoxy-benzyl)-6-(3-methoxy-phenyl)-cyclohexanol
• 1-(4-Chlor-3-trifluormethyl-phenyl)-2-dimethylaminomethyl-6-(3-methoxyphenyl)-cyclohexanol
• 1-(3,5-Dichlor-benzyl)-2-dimethylaminomethyl-6-(3-methoxy-phenyl)-cyclohexanol
• 2-Dimethylaminomethyl-6-(3-methoxy-phenyl)-1-phenylethynylcyclohexanol
• 1-(4-Chlor-phenyl)-2-dimethylaminomethyl-6-(3-methoxy-phenyl)-cyclohexanol
• 2-Dimethylaminomethyl-6-(3-methoxy-phenyl)-1-o-tolyl-cyclohexanol
• 1-(3-Chlor-benzyl)-2-dimethylaminomethyl-6-methyl-cyclohexanol
• 2-Dimethylaminomethyl-1-(2,5-dimethyl-benzyl)-6-methyl-cyclohexanol
• 1-(2-Chlor-6-fluor-benzyl)-2-dimethylaminomethyl-6-methyl-cyclohexanol
• 1-(4-Chlor-benzyl)-2-dimethylaminomethyl-6-methyl-cyclohexanol
• 2-Dimethylaminomethyl-6-methyl-1-(3-methyl-benzyl)-cyclohexanol
• 2-Dimethylaminomethyl-6-methyl-1-(3-trifluormethyl-phenyl)-cyclohexanol
• 1-(2-Chlor-3-fluor-phenyl)-2-dimethylaminomethyl-6-methyl-cyclohexanol
• 2-Dimethylaminomethyl-6-methyl-1-(2-methyl-benzyl)-cyclohexanol
• 2-Dimethylaminomethyl-1-(2-methoxy-phenyl)-6-methyl-cyclohexanol
• 2-Dimethylaminomethyl-1-(3-fluor-benzyl)-6-methyl-cyclohexanol
• 2-Dimethylaminomethyl-1-(4-fluor-benzyl)-6-methyl-cyclohexanol
• 1-(2-Chlor-benzyl)-2-dimethylaminomethyl-6-methyl-cyclohexanol
• 1-(3,5-Dichlor-phenyl)-2-dimethylaminomethyl-6-methyl-cyclohexanol
• 1-(3-Chlor-phenyl)-2-dimethylaminomethyl-6-methyl-cyclohexanol
• 2-Dimethylaminomethyl-1-(3-fluor-phenyl)-6-methyl-cyclohexanol
• 2-Dimethylaminomethyl-1-(3-fluor-4-methoxy-phenyl)-6-methylcyclohexanol
• 2-Dimethylaminomethyl-1-(4-methoxy-phenyl)-6-methyl-cyclohexanol
• 2-Dimethylaminomethyl-6-methyl-1-p-tolyl-cyclohexanol
• 2-Dimethylaminomethyl-6-methyl-1-(3-phenyl-propyl)-cyclohexanol
• 2-Dimethylaminomethyl-1-(3-methoxy-phenyl)-6-methyl-cyclohexanol
• 2-Dimethylaminomethyl-6-methyl-1-thiophen-2-yl-cyclohexanol
• 2-Dimethylaminomethyl-6-methyl-1-phenylethynyl-cyclohexanol
• 2-Dimethylaminomethyl-6-methyl-1-phenethyl-cyclohexanol
• 2-Dimethylaminomethyl-1-(4-fluor-phenyl)-6-methyl-cyclohexanol
• 1-(3-Chlor-benzyl)-2-dimethylaminomethyl-6-(3-methoxy-phenyl)-cyclohexanol
• 2-Dimethylaminomethyl-1-(4-fluor-2-methoxy-phenyl)-6-(3-methoxy-phenyl)-cyclohexanol
• 2-Dimethylaminomethyl-1-(2-methoxy-phenyl)-6-(3-methoxy-phenyl)-cyclohexanol
• 2-Dimethylaminomethyl-1-(4-fluor-benzyl)-6-(3-methoxy-phenyl)-cyclohexanol
• 1-(3-Chlor-phenyl)-2-dimethylaminomethyl-6-(3-methoxy-phenyl)-cyclohexanol
• 2-Dimethylaminomethyl-6-(3-methoxy-phenyl)-1-p-tolyl-cyclohexanol
• 2-Dimethylaminomethyl-6-(3-methoxy-phenyl)-1-(3-phenyl-propyl)-cyclohexanol
• 2-Dimethylaminomethyl-1,6-bis-(3-methoxy-phenyl)-cyclohexanol
• 2-Dimethylaminomethyl-6-(3-methoxy-phenyl)-1-thiophen-2-yl-cyclohexanol
• 2-Dimethylaminomethyl-6-(3-methoxy-phenyl)-1-phenethyl-cyclohexanol
• 3-[3-Dimethylaminomethyl-2-(4-fluor-phenyl)-2-hydroxy-cyclohexyl]-phenol
• 3-(3-Dimethylaminomethyl-2-hydroxy-2-phenyl-cyclohexyl)-phenol
• 3-[2-(4-tert-Butyl-phenyl)-3-dimethylaminomethyl-2-hydroxy-cyclohexyl]-phenol
• 3-(3-Dimethylaminomethyl-2-hydroxy-2-vinyl-cyclohexyl)-phenol
• 1-(4-Chlor-phenyl)-2-dimethylaminomethyl-6-(3-methoxy-phenyl)-cyclohexanol
• 3-Dimethylaminomethyl-2-(3-methoxy-phenyl)-bicyclohexyl-2-ol
• 2-Benzyl-6-dimethylaminomethyl-1-(4-trifluormethyl-phenyl)-cyclohexanol
• 3-(2-Benzyl-6-dimethylaminomethyl-1-hydroxy-cyclohexyl)-phenol
• 3-(2-tert-Butyl-6-dimethylaminomethyl-1-hydroxy-cyclohexyl)-phenol
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate, insbesondere der Hydrochloridsalze.

17. Arzneimittel gemäß einem der Ansprüche 9 bis 16, **dadurch gekennzeichnet, daß** ein enthaltenes substituiertes 1-Aminobutan-3-ol-Derivat als reines Diastereomer und/oder Enantiomer, als Razemat oder als nicht-äquimolare oder äquimolare Mischung der Diastereomere und/oder Enantiomere vorliegt.

18. Verwendung eines substituierten 1-Aminobutan-3-ol-Derivats wie in einem der Ansprüche 9 bis 16 definiert zur Herstellung eines Arzneimittels zur Behandlung von Schmerz, insbesondere von akutem, neuropathischem oder chronischem Schmerz.

19. Verwendung eines substituierten 1-Aminobutan-3-ol-Derivats wie in einem der Ansprüche 9 bis 16 definiert zur Herstellung eines Arzneimittels zur Behandlung von Migräne, Hyperalgesie und Allodynie, insbesondere thermischer Hyperalgesie, mechanischer Hyperalgesie und Allodynie und Kälte Allodynie, oder von inflammatorischem oder postoperativem Schmerz.

20. Verwendung eines substituierten 1-Aminobutan-3-ol-Derivats wie in einem der Ansprüche 9 bis 16 definiert zur Herstellung eines Arzneimittels zur Behandlung von Epilepsie, Hitzewallungen, Beschwerden in der Postmenopause , Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie;
gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

21. Verwendung gemäß einem der Ansprüche 18 - 20, **dadurch gekennzeichnet, daß** ein verwendetes substituiertes 1-Aminobutan-3-ol-Derivat wie in einem der Ansprüche 9 bis 16 definiert als reines Diastereomer und/oder Enantiomer, als Razemat oder als nicht-äquimolare oder äquimolare Mischung der Diastereomere und/oder Enantiomere vorliegt.

22. Verfahren zur Herstellung eines substituierten 1-Aminobutan-3-ol-Derivats gemäß einem der Ansprüche 1 bis 8, in dem ein β-Aminoketon der Formel **Ia**, in dem die Reste R¹ bis R⁴, R⁶ und R⁷ die in Anspruch 1 für Formel **I** beschriebene Bedeutung haben 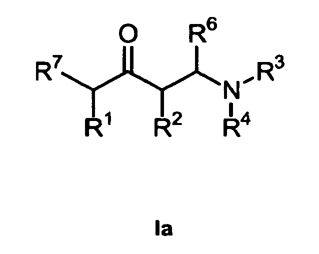 mit einer metallorganischen Verbindung der Formel **III**
R⁵-Z **III**
in der Z MgCl, MgBr, Mgl oder Li bedeutet und R⁵ die in Anspruch 1 für Formel **I** beschriebene Bedeutung hat, zu einer Verbindung der Formel **I** umsetzt.

## Claims

1. Substituted 1-aminobutan-3-ol derivatives of the general formula I 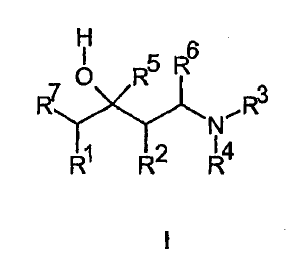 wherein
R¹ and R² together form a (CH₂)₂₋₉ ring that may optionally be substituted with C₁₋₈-alkyl that is branched or unbranched, saturated or unsaturated, unsubstituted or singly or multiply substituted; or aryl that is unsubstituted or singly or multiply substituted,
R³ and R⁴ in each case independently of one another are selected from C₁₋₆-alkyl that is branched or unbranched, saturated or unsaturated, unsubstituted or singly or multiply substituted; C₃₋₆-cycloalkyl that is saturated or unsaturated, unsubstituted or singly or multiply substituted; phenyl, benzyl or phenethyl that is unsubstituted or singly or multiply substituted, or
the radicals R³ and R⁴ together form a ring and denote CH₂CH₂OCH₂CH₂, CH₂CH₂NR²²CH₂CH₂ or (CH₂)₃₋₆
where R²² is selected from H; C₁₋₁₀-alkyl or C₃₋₁₀-cycloalkyl, in each case saturated or unsaturated, branched or unbranched, singly or multiply substituted or unsubstituted; aryl or heteroaryl, in each case singly or multiply substituted or unsubstituted; or aryl, C₃₋₁₀-cycloalkyl or heteroaryl, in each case singly or multiply substituted or unsubstituted, and that is bound via C₁₋₃-alkyl that is saturated or unsaturated;
R⁵ is selected from C₁₋₁₀-alkyl that is saturated or unsaturated, branched or unbranched, singly or multiply substituted or unsubstituted; saturated or unsaturated C₃₋₉-cycloalkyl; aryl, heteroaryl, aryl bound via saturated or unsaturated C₁₋₃-alkyl, C₃₋₁₀-cycloalkyl bound via saturated or unsaturated C₁₋₃-alkyl or heteroaryl bound via saturated or unsaturated C₁₋₃-alkyl, wherein all aryl, heteroaryl and cycloalkyl radicals may in each case independently of one another be unsubstituted or singly or multiply substituted with radicals selected independently of one another from
F, Cl, Br, I, OR¹⁸, SR¹⁸, SO₂R¹⁸ SO₂OR¹⁸, CN, COOR¹⁸, NR¹⁹R²⁰; C₁₋₁₀-alkyl that is saturated or unsaturated, branched or unbranched, singly or multiply substituted or unsubstituted; C₃₋₉-cycloalkyl that is saturated or unsaturated, unsubstituted or singly or multiply substituted; aryl or heteroaryl, in each case unsubstituted or singly or multiply substituted; or aryl, C₃₋₉-cycloalkyl or heteroaryl, in each case unsubstituted or singly or multiply substituted, and that is bound via saturated or unsaturated C₁₋₃-alkyl;
where R¹⁸ is selected from H; C₁₋₁₀-alkyl that is saturated or unsaturated, branched or unbranched, unsubstituted or singly or multiply substituted; C₃₋₉-cycloalkyl that is saturated or unsaturated, unsubstituted or singly or multiply substituted; aryl or heteroaryl that is unsubstituted or singly or multiply substituted; or aryl, C₃₋₉-cycloalkyl or heteroaryl, in each case unsubstituted or singly or multiply substituted, and that is bound via saturated or unsaturated C₁₋₃-alkyl;
R¹⁹ and R²⁰ are selected independently of one another from H; C₁₋₁₀-alkyl that is saturated or unsaturated, branched or unbranched, unsubstituted or singly or multiply substituted; C₃₋₉-cycloalkyl that is saturated or unsaturated, unsubstituted or singly or multiply substituted; aryl or heteroaryl in each case unsubstituted or singly or multiply substituted; or aryl, C₃₋₉-cycloalkyl or heteroaryl, in each case unsubstituted or singly or multiply substituted, and that is bound via saturated or unsaturated C₁₋₃-alkyl; or R¹⁹ and R²⁰ together form CH₂CH₂OCH₂CH₂, CH₂CH₂NR²¹CH₂CH₂ or (CH₂)₃₋₆,
where R²¹ is selected from H, C₁₋₁₀-alkyl that is saturated or unsaturated, branched or unbranched, unsubstituted or singly or multiply substituted;
R⁶ is selected from H; aryl or heteroaryl in each case unsubstituted or singly or multiply substituted; and
R⁷ is selected from halogen, CF₃; C₃₋₉-cycloalkyl that is saturated or unsaturated, unsubstituted or singly or multiply substituted; aryl, heteroaryl in each case unsubstituted or singly or multiply substituted; aryl, C₃₋₉-cycloalkyl or heteroaryl, in each case unsubstituted or singly or multiply substituted, and that is bound via saturated or unsaturated C₁₋₃-alkyl
optionally in the form of their racemates, their pure stereoisomers, in particular enantiomers or diastereomers, or in the form of mixtures of the stereoisomers, in particular of the enantiomers or diastereomers, in an arbitrary mixture ratio; in the illustrated form or in the form of their acids or their bases, or in the form of their salts, in particular the physiologically acceptable salts, or in the form of their solvates, in particular the hydrates.

2. Substituted 1-aminobutan-3-ol derivatives according to claim 1, **characterised in that**
R⁶ is selected from H or heteroaryl or is preferably a radical according to formula II 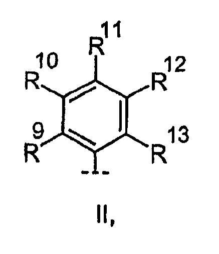 where R⁹ to R¹³ in each case independently of one another are selected from H, F, Cl, Br, I, CF₃, OH, OR¹⁴, OCF₃, SR¹⁴, SO₂CH₃, SO₂CF₃; C₁₋₆-alkyl that is branched or unbranched, saturated or unsaturated, unsubstituted or singly or multiply substituted; phenyl that is unsubstituted or singly or multiply substituted; CN, COOR¹⁴, NO₂ or
R⁹ and R¹⁰ or R¹⁰ and R¹¹ together form an OCH₂O or OCH₂CH₂O ring, and
R¹⁴ is selected from C₁₋₆-alkyl that is branched or unbranched, saturated or unsaturated, unsubstituted or singly or multiply substituted; phenyl, benzyl, phenethyl or thiophene, in each case unsubstituted or singly or multiply substituted.

3. Substituted 1-aminobutan-3-ol derivatives according to either of claims 1 or 2, **characterised in that**
R¹ and R² together form a (H₂)₂₋₅ ring that may optionally be substituted by C₁₋₆-alkyl that is branched or unbranched, saturated or unsaturated, unsubstituted or singly or multiply substituted; or phenyl that is unsubstituted or singly or multiply substituted; but is preferably unsubstituted.

4. Substituted 1-aminobutan-3-ol derivatives according to any one of claims 1 to 3, **characterised in that**
R³ and R⁴ in each case independently of one another are selected from C₁₋₆-alkyl that is branched or unbranched, saturated or unsaturated, unsubstituted or singly or multiply substituted; preferably both are CH₃;
or the radicals R³ and R⁴ together form a ring and denote CH₂CH₂NR²²CH₂CH₂ or (CH₂)₃₋₆, and in particular together denote (CH₂)₄₋₅ or CH₂CH₂NR²²CH₂CH₂, where R²² is selected from H or C₁₋₆-alkyl that is saturated, branched or unbranched and unsubstituted; in particular H or CH₃.

5. Substituted 1-aminobutan-3-ol derivatives according to any one of claims 1 to 4, **characterised in that**
R⁵ is selected from C₁₋₆-alkyl that is saturated or unsaturated, branched or unbranched, singly or multiply substituted or unsubstituted; C₅₋₆-cycloalkyl, phenyl, thiophenyl, furyl, benzofuranyl, benzothiophenyl, pyrrolyl, pyridinyl, pyrimidinyl, quinolinyl, isoquinolinyl, quinazolinyl, phenyl bonded via saturated or unsaturated C₁₋₃-alkyl, C₅₋₆-cycloalkyl bonded via saturated or unsaturated C₁₋₃-alkyl, or thiophenyl, furyl, benzofuranyl, benzothiophenyl, pyrrolyl, pyridinyl, pyrimidinyl, quinolinyl, isoquinolinyl, quinazolinyl bonded via saturated or unsaturated C₁₋₃-alkyl, wherein all aryl, heteroaryl and cycloalkyl radicals may in each case independently of one another be unsubstituted or singly or multiply substituted,
preferably
R⁵ is selected from phenyl or thiophenyl that is unsubstituted or singly or multiply substituted, preferably with F, Cl, Br, I, OH, O-C₁₋₄-alkyl, C₁₋₆-alkyl, NH₂ and/or SH; or phenyl bonded via saturated or unsaturated C₁₋₃-alkyl and that is unsubstituted or singly or multiply substituted, preferably with F, Cl, Br, I, OH, O-C₁₋₄-alkyl, C₁₋₆-alkyl, NH₂ and/or SH,
in particular
R⁵ is selected from phenyl that is unsubstituted or singly or multiply substituted, preferably with F, Cl, Br, I, OH, OCH₃, OC₂H₅, OC₃H₇, SH, CH₃, C₂H₅, C₃H₇ and/or C₄H₉.

6. Substituted 1-aminobutan-3-ol derivatives according to any one of claims 1 to 5, **characterised in that**
R⁷ is selected from C₅₋₇-cycloalkyl that is saturated or unsaturated, unsubstituted or singly or multiply substituted, preferably cyclohexyl; phenyl that is unsubstituted or singly or multiply substituted; phenyl that is bound via saturated or unsaturated C₁₋₃-alkyl, in each case unsubstituted or singly or multiply substituted;
preferably R⁷ is selected from phenyl that is unsubstituted or singly or multiply substituted; phenyl bound via saturated or unsaturated C₁₋₃-alkyl, in each case unsubstituted or singly or multiply substituted.

7. Substituted 1-aminobutan-3-ol derivatives according to any one of claims 1 to 6, **characterised in that** if R⁵ and/or R⁷ is/are selected from aryl, C₃₋₉-cycloalkyl or heteroaryl bound via saturated or unsaturated C₁₋₃-alkyl, then the C₁₋₃-alkyl bound via the aryl, heteroaryl or cycloalkyl is selected from:
- CH₂-, -C₂H₄-, -C₃H₆-, -C≡C-, -CH=CH-, -CH=CH-CH₂-, -CH₂-CH=CH-, -C≡C-CH₂- or -CH₂-C≡C-, preferably -CH₂-, -C₂H₄- or -C≡C- .

8. Substituted 1-aminobutan-3-ol derivatives according to any one of claims 1 to 7, **characterised in that** they are selected from the following group:
• 2-benzyl-1-(2,4-dichlorobenzyl)-6-dimethylaminomethylcyclohexanol
• 2-benzyl-1-(3-chlorobenzyl)-6-dimethylaminomethylcyclohexanol
• 2-benzyl-6-dimethylaminomethyl-1-(2,5-dimethylbenzyl)-cyclohexanol
• 2-benzyl-1-(2-chloro-6-fluorobenzyl)-6-dimethylaminomethylcyclohexanol
• 2-benzyl-1-(4-chlorobenzyl)-6-dimethylaminomethylcyclohexanol
• 2-benzyl-6-dimethylaminomethyl-1-(3-trifluoromethylphenyl)-cyclohexanol
• 2-benzyl-6-dimethylaminomethyl-1-(2-methylbenzyl)-cyclohexanol
• 2-benzyl-6-dimethylaminomethyl-1-(2-methoxyphenyl)-cyclohexanol
• 2-benzyl-1-(4-chloro-3-trifluoromethylphenyl)-6-dimethylaminomethylcyclohexanol
• 2-benzyl-1-(2-chlorobenzyl)-6-dimethylaminomethylcyclohexanol
• 2-benzyl-1-(3,5-dichlorophenyl)-6-dimethylaminomethylcyclohexanol
• 2-benzyl-1-(3-chlorophenyl)-6-dimethylaminomethylcyclohexanol
• 2-benzyl-6-dimethylaminomethyl-1-(3-fluorophenyl)-cyclohexanol
• 2-benzyl-6-dimethylaminomethyl-1-(5-fluoro-2-methoxy-phenyl)-cyclohexanol
• 2-benzyl-1-cyclohexylmethyl-6-dimethylaminomethylcyclohexanol
• 2-benzyl-6-dimethylaminomethyl-1-(4-methoxyphenyl)-cyclohexanol
• 2-benzyl-6-dimethylaminomethyl-1-p-tolyl-cyclohexanol
• 2-benzyl-6-dimethylaminomethyl-1-(3-phenylpropyl)-cyclohexanol
• 2-benzyl-6-dimethylaminomethyl-1-(3-methoxyphenyl)-cyclohexanol
• 2-benzyl-6-dimethylaminomethyl-1-thiophen-2-yl-cyclohexanol
• 2-benzyl-6-dimethylaminomethyl-1-phenylethynylcyclohexanol
• 2-benzyl-6-dimethylaminomethyl-1-(4-fluorophenyl)-cyclohexanol
• 2-benzyl-6-dimethylaminomethylbicyclohexyl-1-ol
• 2-benzyl-6-dimethylaminomethyl-1-m-tolyl-cyclohexanol
• 2-benzyl-1-(4-tert-butylphenyl)-6-dimethylaminomethylcyclohexanol
• 2-benzyl-6-dimethylaminomethyl-1-vinylcyclohexanol
• 2-benzyl-6-dimethylaminomethyl-1-o-tolyl-cyclohexanol
• 2-benzyl-6-dimethylaminomethyl-1-(4-fluoro-3-methylphenyl)-cyclohexanol
• 1,2-dibenzyl-6-dimethylaminomethylcyclohexanol
• 2-benzyl-1-(4-chlorophenyl)-6-dimethylaminomethylcyclohexanol
• 2-benzyl-6-dimethylaminomethyl-1-phenylcyclohexanol
• 2-dimethylaminomethyl-1-(2,5-dimethylphenyl)-6-(3-methoxyphenyl)-cyclohexanol
• 1-cyclohexylmethyl-2-dimethylaminomethyl-6-(3-methoxyphenyl)-cyclohexanol
• 1-(2,4-dichlorobenzyl)-2-dimethylaminomethyl-6-(3-methoxyphenyl)-cyclohexanol
• 2-dimethylaminomethyl-6-(3-methoxyphenyl)-1-phenylethynylcyclohexanol
• 2-dimethylaminomethyl-6-(3-methoxyphenyl)-1-o-tolylcyclohexanol
• 1-(4-chlorophenyl)-2-dimethylaminomethyl-6-(3-methoxyphenyl)-cyclohexanol
• 2-dimethylaminomethyl-1-(3-fluorophenyl)-6-(3-methoxyphenyl)-cyclohexanol
• 2-dimethylaminomethyl-1-(3-fluoro-4-methoxyphenyl)-6-(3-methoxyphenyl)-cyclohexanol
• 1-(3-chlorobenzyl)-2-dimethylaminomethyl-6-(3-methoxyphenyl)-cyclohexanol
• 2-dimethylaminomethyl-1-(3-methoxybenzyl)-6-(3-methoxyphenyl)-cyclohexanol
• 1-(4-chloro-3-trifluoromethylphenyl)-2-dimethylaminomethyl-6-(3-methoxyphenyl)-cyclohexanol
• 1-(3,5-dichlorobenzyl)-2-dimethylaminomethyl-6-(3-methoxyphenyl)-cyclohexanol
• 2-dimethylaminomethyl-6-(3-methoxyphenyl)-1-phenylethynylcyclohexanol
• 1-(4-chlorophenyl)-2-dimethylaminomethyl-6-(3-methoxyphenyl)-cyclohexanol
• 2-dimethylaminomethyl-6-(3-methoxyphenyl)-1-o-tolylcyclohexanol
• 1-(3-chlorobenzyl)-2-dimethylaminomethyl-6-(3-methoxyphenyl)-cyclohexanol
• 2-dimethylaminomethyl-1-(4-fluoro-2-methoxyphenyl)-6-(3-methoxyphenyl)-cyclohexanol
• 2-dimethylaminomethyl-1-(2-methoxyphenyl)-6-(3-methoxyphenyl)-cyclohexanol
• 2-dimethylaminomethyl-1-(4-fluorobenzyl)-6-(3-methoxy-phenyl)-cyclohexanol
• 1-(3-chlorophenyl)-2-dimethylaminomethyl-6-(3-methoxy-phenyl)-cyclohexanol
• 2-dimethylaminomethyl-6-(3-methoxyphenyl)-1-p-tolylcyclohexanol
• 2-dimethylaminomethyl-6-(3-methoxyphenyl)-1-(3-phenylpropyl)-cyclohexanol
• 2-dimethylaminomethyl-1,6-bis-(3-methoxyphenyl)-cyclohexanol
• 2-dimethylaminomethyl-6-(3-methoxyphenyl)-1-thiophen-2-yl-cyclohexanol
• 2-dimethylaminomethyl-6-(3-methoxyphenyl)-1-phenethyl-cyclohexanol
• 3-[3-dimethylaminomethyl-2-(4-fluorophenyl)-2-hydroxycyclohexyl]-phenol
• 3-(3-dimethylaminomethyl-2-hydroxy-2-phenylcyclohexyl)-phenol
• 3-[2-(4-tert-butylphenyl)-3-dimethylaminomethyl-2-hydroxycyclohexyl]phenol
• 3-(3-dimethylaminomethyl-2-hydroxy-2-vinyl-cyclohexyl)-phenol
• 1-(4-chlorophenyl)-2-dimethylaminomethyl-6-(3-methoxyphenyl)-cyclohexanol
• 3-dimethylaminomethyl-2-(3-methoxyphenyl)-bicyclo-hexyl-2-ol
• 2-benzyl-6-dimethylaminomethyl-1-(4-trifluoromethylphenyl)-cyclohexanol
• 3-(2-benzyl-6-dimethylaminomethyl-1-hydroxycyclohexyl)-phenol
• 3-(2-tert-butyl-6-dimethylaminomethyl-1-hydroxycyclohexyl)-phenol
in the form of their pure stereoisomers, in particular enantiomers or diastereomers.

9. Medicament containing at least one substituted 1-aminobutan-3-ol derivative of the general formula I 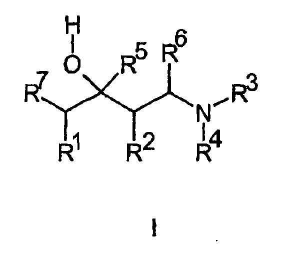 wherein
R¹ and R² together form a (CH₂)₂₋₉ ring that may optionally be substituted with C₁₋₈-alkyl that is branched or unbranched, saturated or unsaturated, unsubstituted or singly or multiply substituted; or aryl that is unsubstituted or singly or multiply substituted,
R³ and R⁴ in each case independently of one another are selected from C₁₋₆-alkyl that is branched or unbranched, saturated or unsaturated, unsubstituted or singly or multiply substituted; C₃₋₆-cycloalkyl that is saturated or unsaturated, unsubstituted or singly or multiply substituted; phenyl, benzyl or phenethyl that is unsubstituted or singly or multiply substituted, or
the radicals R³ and R⁴ together form a ring and denote CH₂CH₂OCH₂CH₂, CH₂CH₂NR²²CH₂CH₂ or (CH₂)₃₋₆
where R²² is selected from H; C₁₋₁₀-alkyl or C₃₋₁₀-cycloalkyl, in each case saturated or unsaturated, branched or unbranched, singly or multiply substituted or unsubstituted; aryl or heteroaryl, in each case singly or multiply substituted or unsubstituted; or aryl, C₃₋₁₀-cycloalkyl or heteroaryl, in each case singly or multiply substituted or unsubstituted, and that is bound via C₁₋₃-alkyl that is saturated or unsaturated;
R⁵ is selected from C₁₋₁₀-alkyl that is saturated or unsaturated, branched or unbranched, singly or multiply substituted or unsubstituted; saturated or unsaturated C₃₋₉-cycloalkyl; aryl, heteroaryl, aryl bound via saturated or unsaturated C₁₋₃-alkyl, C₃₋₁₀-cycloalkyl bound via saturated or unsaturated C₁₋₃-alkyl or heteroaryl bound via saturated or unsaturated C₁₋₃-alkyl, wherein all aryl, heteroaryl and cycloalkyl radicals may in each case independently of one another be unsubstituted or singly or multiply substituted with radicals selected independently of one another from
F, Cl, Br, I, OR¹⁸, SR¹⁸, SO₂R¹⁸, SO₂OR¹⁸, CN, COOR¹⁸, NR¹⁹R²⁰ ; C₁₋₁₀-alkyl that is saturated or unsaturated, branched or unbranched, singly or multiply substituted or unsubstituted; C₃₋₉-cycloalkyl that is saturated or unsaturated, unsubstituted or singly or multiply substituted; aryl or heteroaryl, in each case unsubstituted or singly or multiply substituted; or aryl, C₃₋₉-cycloalkyl or heteroaryl, in each case unsubstituted or singly or multiply substituted, and that is bound via saturated or unsaturated C₁₋₃-alkyl;
where R¹⁸ is selected from H; C₁₋₁₀-alkyl that is saturated or unsaturated, branched or unbranched, unsubstituted or singly or multiply substituted; C₃₋₉-cycloalkyl that is saturated or unsaturated, unsubstituted or singly or multiply substituted; aryl or heteroaryl that is unsubstituted or singly or multiply substituted; or aryl, C₃₋₉-cycloalkyl or heteroaryl, in each case unsubstituted or singly or multiply substituted, and that is bound via saturated or unsaturated C₁₋₃-alkyl;
R¹⁹ and R²⁰ are selected independently of one another from H; C₁₋₁₀-alkyl that is saturated or unsaturated, branched or unbranched, unsubstituted or singly or multiply substituted; C₃₋₉-cycloalkyl that is saturated or unsaturated, unsubstituted or singly or multiply substituted; aryl or heteroaryl in each case unsubstituted or singly or multiply substituted; or aryl, C₃₋₉-cycloalkyl or heteroaryl, in each case unsubstituted or singly or multiply substituted, and that is bound via saturated or unsaturated C₁₋₃-alkyl;
or R¹⁹ and R²⁰ together form CH₂CH₂OCH₂CH₂, CH₂CH₂NR²¹CH₂CH₂ or (CH₂)₃₋₆,
where R²¹ is selected from H, C₁₋₁₀-alkyl that is saturated or unsaturated, branched or unbranched, unsubstituted or singly or multiply substituted;
R⁶ is selected from H; aryl or heteroaryl in each case unsubstituted or singly or multiply substituted; and
R⁷ is selected from halogen, CF₃; C₁-C₆-alkyl that is branched or unbranched, saturated or unsaturated, unsubstituted or singly or multiply substituted; C₃₋₉-cycloalkyl that is saturated or unsaturated, unsubstituted or singly or multiply substituted; aryl, heteroaryl in each case unsubstituted or singly or multiply substituted; aryl, C₃₋₉-cycloalkyl or heteroaryl, in each case unsubstituted or singly or multiply substituted, and that is bound via saturated or unsaturated C₁₋₃-alkyl
optionally in the form of their racemates, their pure stereoisomers, in particular enantiomers or diastereomers, or in the form of mixtures of the stereoisomers, in particular of the enantiomers or diastereomers, in an arbitrary mixture ratio; in the illustrated form or in the form of their acids or their bases, or in the form of their salts, in particular the physiologically acceptable salts, or in the form of their solvates, in particular the hydrates.
as well as optionally suitable additives and/or auxiliary substances and/or optionally further active constituents.

10. Medicament according to claim 9, **characterised in that** R⁶ is selected from H or heteroaryl or is preferably a radical according to formula II 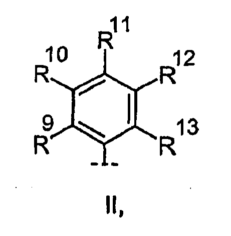 where R⁹ to R¹³ in each case independently of one another are selected from H, F, Cl, Br, I, CF₃, OH, OR¹⁴, OCF₃, SR¹⁴, SO₂CH₃, SO₂CF₃; C₁₋₆-alkyl that is branched or unbranched, saturated or unsaturated, unsubstituted or singly or multiply substituted; phenyl that is unsubstituted or singly or multiply substituted; CN, COOR¹⁴, NO₂ or R⁹ and R¹⁰ or R¹⁰ and R¹¹ together form an OCH₂O or OCH₂CH₂O ring, and
R¹⁴ is selected from C₁₋₆-alkyl that is branched or unbranched, saturated or unsaturated, unsubstituted or singly or multiply substituted; phenyl, benzyl, phenethyl or thiophene, in each case unsubstituted or singly or multiply substituted.

11. Medicament according to claim 9 or 10, **characterised in that**
R¹ and R² together form a (CH₂)₂₋₅ ring that may optionally be substituted by C₁₋₆-alkyl that is branched or unbranched, saturated or unsaturated, unsubstituted or singly or multiply substituted; or phenyl that is unsubstituted or singly or multiply substituted; but is preferably unsubstituted.

12. Medicament according to one or more of claims 9 to 11, **characterised in that**
R³ and R⁴ in each case independently of one another are selected from C₁₋₆-alkyl that is branched or unbranched, saturated or unsaturated, unsubstituted or singly or multiply substituted; preferably both are CH₃;
or the radicals R³ and R⁴ together form a ring and denote CH₂CH₂NR²²CH₂CH₂ or (CH₂)₃₋₆, and in particular together denote (CH₂)₄₋₅ or CH₂CH₂NR²²CH₂CH₂, where R²² is selected from H or C₁₋₆-alkyl that is saturated, branched or unbranched and unsubstituted; in particular H or CH₃ .

13. Medicament according to one or more of claims 9 to 12, **characterised in that**
R⁵ is selected from C₁₋₆-alkyl that is saturated or unsaturated, branched or unbranched, singly or multiply substituted or unsubstituted; C₅₋₆-cycloalkyl, phenyl, thiophenyl, furyl, benzofuranyl, benzothiophenyl, pyrrolyl, pyridinyl, pyrimidinyl, quinolinyl, isoquinolinyl, quinazolinyl, phenyl bonded via saturated or unsaturated C₁₋₃-alkyl, C₅₋₆-cycloalkyl bonded via saturated or unsaturated C₁₋₃-alkyl, or thiophenyl, furyl, benzofuranyl, benzothiophenyl, pyrrolyl, pyridinyl, pyrimidinyl, quinolinyl, isoquinolinyl, quinazolinyl bonded via saturated or unsaturated C₁₋₃-alkyl, wherein all aryl, heteroaryl and cycloalkyl radicals may in each case independently of one another be unsubstituted or singly or multiply substituted,
preferably
R⁵ is selected from phenyl or thiophenyl that is unsubstituted or singly or multiply substituted, preferably with F, Cl, Br, I, OH, O-C₁₋₄-alkyl, C₁₋₆-alkyl, NH₂ and/or SH; or phenyl bonded via saturated or unsaturated C₁₋₃-alkyl and that is unsubstituted or singly or multiply substituted, preferably with F, Cl, Br, I, OH, O-C₁₋₄-alkyl, C₁₋₆-alkyl, NH₂ and/or SH,
in particular
R⁵ is selected from phenyl that is unsubstituted or singly or multiply substituted, preferably with F, Cl, Br, I, OH, OCH₃, OC₂H₅, OC₃H₇, SH, CH₃, C₂H₅, C₃H₇ and/or C₄H₉.

14. Medicament according to one or more of claims 9 to 13, **characterised in that**
R⁷ is selected from C₁-C₆-alkyl that is branched or unbranched, saturated or unsaturated, unsubstituted or singly or multiply substituted; C₅₋₇-cycloalkyl that is saturated or unsaturated, unsubstituted or singly or multiply substituted, preferably cyclohexyl; phenyl that is unsubstituted or singly or multiply substituted; phenyl that is bound via saturated or unsaturated C₁₋₃-alkyl, in each case unsubstituted or singly or multiply substituted;
preferably R⁷ is selected from phenyl that is unsubstituted or singly or multiply substituted; phenyl bound via saturated or unsaturated C₁₋₃-alkyl, in each case unsubstituted or singly or multiply substituted.

15. Medicament according to one or more of claims 9 to 14, **characterised in that** if R⁵ and/or R⁷ is/are selected from aryl, C₃₋₉-cycloalkyl or heteroaryl bound via saturated or unsaturated C₁₋₃-alkyl, then the C₁₋₃-alkyl bound via the aryl, heteroaryl or cycloalkyl is selected from:
- CH₂-, -C₂H₄-, -C₃H₆-, -C≡C-, -CH=CH-, -CH=CH-CH₂-, -CH₂-CH=CH-, -C≡C-CH₂- or -CH₂-C≡C-, preferably -CH-, -C₂H₄- or -C≡C-.

16. Medicament according to one or more of claims 9-15, **characterised in that** the substituted 1-aminobutan-3-ol derivatives are selected from the following group:
• 2-benzyl-1-(2,4-dichlorobenzyl)-6-dimethylaminomethylcyclohexanol
• 2-benzyl-1-(3-chlorobenzyl)-6-dimethylaminomethylcyclohexanol
• 2-benzyl-6-dimethylaminomethyl-1-(2,5-dimethylbenzyl)-cyclohexanol
• 2-benzyl-1-(2-chloro-6-fluorobenzyl)-6-dimethylaminomethylcyclohexanol
• 2-benzyl-1-(4-chlorobenzyl)-6-dimethylaminomethylcyclohexanol
• 2-benzyl-6-dimethylaminomethyl-1-(3-trifluoromethylphenyl)-cyclohexanol
• 2-benzyl-6-dimethylaminomethyl-1-(2-methylbenzyl)-cyclohexanol
• 2-benzyl-6-dimethylaminomethyl-1-(2-methoxyphenyl)-cyclohexanol
• 2-benzyl-1-(4-chloro-3-trifluoromethylphenyl)-6-dimethylaminomethylcyclohexanol
• 2-benzyl-1-(2-chlorobenzyl)-6-dimethylaminomethylcyclohexanol
• 2-benzyl-1-(3,5-dichlorophenyl)-6-dimethylaminomethylcyclohexanol
• 2-benzyl-1-(3-chlorophenyl)-6-dimethylaminomethylcyclohexanol
• 2-benzyl-6-dimethylaminomethyl-1-(3-fluorophenyl)-cyclohexanol
• 2-benzyl-6-dimethylaminomethyl-1-(5-fluoro-2-methoxy-phenyl)-cyclohexanol
• 2-benzyl-1-cyclohexylmethyl-6-dimethylaminomethylcyclohexanol
• 2-benzyl-6-dimethylaminomethyl-1-(4-methoxyphenyl)-cyclohexanol
• 2-benzyl-6-dimethylaminomethyl-1-p-tolyl-cyclohexanol
• 2-benzyl-6-dimethylaminomethyl-1-(3-phenylpropyl)-cyclohexanol
• 2-benzyl-6-dimethylaminomethyl-1-(3-methoxyphenyl)-cyclohexanol
• 2-benzyl-6-dimethylaminomethyl-1-thiophen-2-yl-cyclohexanol
• 2-benzyl-6-dimethylaminomethyl-1-phenylethynylcyclohexanol
• 2-benzyl-6-dimethylaminomethyl-1-(4-fluorophenyl)-cyclohexanol
• 2-benzyl-6-dimethylaminomethylbicyclohexyl-1-ol
• 2-benzyl-6-dimethylaminomethyl-1-m-tolyl-cyclohexanol
• 2-benzyl-1-(4-tert-butylphenyl)-6-dimethylaminomethylcyclohexanol
• 2-benzyl-6-dimethylaminomethyl-1-vinyl-cyclohexanol
• 2-benzyl-6-dimethylaminomethyl-1-o-tolyl-cyclohexanol
• 2-benzyl-6-dimethylaminomethyl-1-(4-fluoro-3-methylphenyl)-cyclohexanol
• 1,2-dibenzyl-6-dimethylaminomethylcyclohexanol
• 2-benzyl-1-(4-chlorophenyl)-6-dimethylaminomethylcyclohexanol
• 2-benzyl-6-dimethylaminomethyl-1-phenylcyclohexanol
• 2-dimethylaminomethyl-1-(2,5-dimethylphenyl)-6-(3-methoxyphenyl)-cyclohexanol
• 1-cyclohexylmethyl-2-dimethylaminomethyl-6-(3-methoxyphenyl)-cyclohexanol
• 1-(2,4-dichlorobenzyl)-2-dimethylaminomethyl-6-(3-methoxyphenyl)-cyclohexanol
• 2-dimethylaminomethyl-6-(3-methoxyphenyl)-1-phenylethynylcyclohexanol
• 2-dimethylaminomethyl-6-(3-methoxyphenyl)-1-o-tolylcyclohexanol
• 1-(4-chlorophenyl)-2-dimethylaminomethyl-6-(3-methoxyphenyl)-cyclohexanol
• 2-dimethylaminomethyl-1-(3-fluorophenyl)-6-(3-methoxyphenyl)-cyclohexanol
• 2-dimethylaminomethyl-1-(3-fluoro-4-methoxyphenyl)-6-(3-methoxyphenyl)-cyclohexanol
• 1-(3-chlorobenzyl)-2-dimethylaminomethyl-6-(3-methoxyphenyl)-cyclohexanol
• 2-dimethylaminomethyl-1-(3-methoxybenzyl)-6-(3-methoxyphenyl)-cyclohexanol
• 1-(4-chloro-3-trifluoromethylphenyl)-2-dimethylaminomethyl-6-(3-methoxyphenyl)-cyclohexanol
• 1-(3,5-dichlorobenzyl)-2-dimethylaminomethyl-6-(3-methoxyphenyl)-cyclohexanol
• 2-dimethylaminomethyl-6-(3-methoxyphenyl)-1-phenylethynylcyclohexanol
• 1-(4-chlorophenyl)-2-dimethylaminomethyl-6-(3-methoxyphenyl)-cyclohexanol
• 2-dimethylaminomethyl-6-(3-methoxyphenyl)-1-o-tolylcyclohexanol
• 1-(3-chlorobenzyl)-2-dimethylaminomethyl-6-methylcyclohexanol
• 2-dimethylaminomethyl-1-(2,5-dimethylbenzyl)-6-methylcyclohexanol
• 1-(2-chloro-6-fluorobenzyl)-2-dimethylaminomethyl-6-methylcyclohexanol
• 1-(4-chlorobenzyl)-2-dimethylaminomethyl-6-methylcyclohexanol
• 2-dimethylaminomethyl-6-methyl-1-(3-methylbenzyl)-cyclohexanol
• 2-dimethylaminomethyl-6-methyl-1-(3-trifluoromethylphenyl)-cyclohexanol
• 1-(2-chloro-3-fluorophenyl)-2-dimethylaminomethyl-6-methylcyclohexanol
• 2-dimethylaminomethyl-6-methyl-1-(2-methylbenzyl)-cyclohexanol
• 2-dimethylaminomethyl-1-(2-methoxyphenyl)-6-methylcyclohexanol
• 2-dimethylaminomethyl-1-(3-fluorobenzyl)-6-methylcyclohexanol
• 2-dimethylaminomethyl-1-(4-fluorobenzyl)-6-methylcyclohexanol
• 1-(2-chlorobenzyl)-2-dimethylaminomethyl-6-methylcyclohexanol
• 1-(3,5-dichlorophenyl)-2-dimethylaminomethyl-6-methylcyclohexanol
• 1-(3-chlorophenyl)-2-dimethylaminomethyl-6-methylcyclohexanol
• 2-dimethylaminomethyl-1-(3-fluorophenyl)-6-methylcyclohexanol
• 2-dimethylaminomethyl-1-(3-fluoro-4-methoxyphenyl)-6-methylcyclohexanol
• 2-dimethylaminomethyl-1-(4-methoxyphenyl)-6-methylcyclohexanol
• 2-dimethylaminomethyl-6-methyl-1-p-tolyl-cyclohexanol
• 2-dimethylaminomethyl-6-methyl-1-(3-phenylpropyl)-cyclohexanol
• 2-dimethylaminomethyl-1-(3-methoxyphenyl)-6-methyl-cyclohexanol
• 2-dimethylaminomethyl-6-methyl-1-thiophen-2-yl-cyclohexanol
• 2-dimethylaminomethyl-6-methyl-1-phenylethynylcyclohexanol
• 2-dimethylaminomethyl-6-methyl-1-phenethylcyclohexanol
• 2-dimethylaminomethyl-1-(4-fluorophenyl)-6-methylcyclohexanol
• 1-(3-chlorobenzyl)-2-dimethylaminomethyl-6-(3-methoxyphenyl)-cyclohexanol
• 2-dimethylaminomethyl-1-(4-fluoro-2-methoxyphenyl)-6-(3-methoxyphenyl)-cyclohexanol
• 2-dimethylaminomethyl-1-(2-methoxyphenyl)-6-(3-methoxyphenyl)-cyclohexanol
• 2-dimethylaminomethyl-1-(4-fluorobenzyl)-6-(3-methoxy-phenyl)-cyclohexanol
• 1-(3-chlorophenyl)-2-dimethylaminomethyl-6-(3-methoxy-phenyl)-cyclohexanol
• 2-dimethylaminomethyl-6-(3-methoxyphenyl)-1-p-tolylcyclohexanol
• 2-dimethylaminomethyl-6-(3-methoxyphenyl)-1-(3-phenylpropyl)-cyclohexanol
• 2-dimethylaminomethyl-1,6-bis-(3-methoxyphenyl)-cyclohexanol
• 2-dimethylaminomethyl-6-(3-methoxyphenyl)-1-thiophen-2-yl-cyclohexanol
• 2-dimethylaminomethyl-6-(3-methoxyphenyl)-1-phenethyl-cyclohexanol
• 3-[3-dimethylaminomethyl-2-(4-fluorophenyl)-2-hydroxycyclohexyl]-phenol
• 3-(3-dimethylaminomethyl-2-hydroxy-2-phenylcyclohexyl)-phenol
• 3-[2-(4-tert-butylphenyl)-3-dimethylaminomethyl-2-hydroxycyclohexyl]phenol
• 3-(3-dimethylaminomethyl-2-hydroxy-2-vinylcyclohexyl)-phenol
• 1-(4-chlorophenyl)-2-dimethylaminomethyl-6-(3-methoxyphenyl)-cyclohexanol
• 3-dimethylaminomethyl-2-(3-methoxyphenyl)-bicyclo-hexyl-2-ol
• 2-benzyl-6-dimethylaminomethyl-1-(4-trifluoromethylphenyl)-cyclohexanol
• 3-(2-benzyl-6-dimethylaminomethyl-1-hydroxycyclohexyl)-phenol
• 3-(2-tert-butyl-6-dimethylaminomethyl-1-hydroxycyclohexyl)-phenol
optionally in the form of their racemates, their pure stereoisomers, in particular enantiomers or diastereomers, or in the form of mixtures of the stereoisomers, in particular of the enantiomers or diastereomers, in an arbitrary mixture ratio; in the represented form or in the form of their acids or their bases or in the form of their salts, in particular of the physiologically acceptable salts, or in the form of their solvates, in particular the hydrates, especially the hydrochloride salts.

17. Medicament according to any one of claims 9 to 16, **characterised in that** a contained substituted 1-aminobutan-3-ol derivative is present as a pure diastereomer and/or enantiomer, as a racemate, or as a non-equimolar or equimolar mixture of the diastereomers and/or enantiomers.

18. Use of a substituted 1-aminobutan-3-ol derivative as defined in any one of claims 9 to 16 for the production of a medicament for treating pain, in particular acute, neuropathic or chronic pain.

19. Use of a substituted 1-aminobutan-3-ol derivative as defined in any one of claims 9 to 16 for the production of a medicament for treating migraines, hyperalgesia and allodynia, in particular thermal • hyperalgesia, mechanical hyperalgesia and allodynia and cold-induced allodynia, or inflammatory or postoperative pain.

20. Use of a substituted 1-aminobutan-3-ol derivative as defined in any one of claims 9 to 16 for the production of a medicament for treating epilepsy, hot flushes, post-menopausal symptoms, amyotropic lateral sclerosis (ALS), reflex sympathetic dystrophy (RSD), spastic paralysis, restless leg syndrome, acquired nystagmus; psychiatric or neuropathological disorders such as bipolar disorders, anxiety, panic attacks, mood fluctuations, manic behaviour, depression, manic-depressive behaviour; painful diabetic neuropathy, symptoms and pain due to multiple sclerosis or Parkinson's disease, neurodegenerative diseases such as Alzheimer's disease, Huntington's disease, Parkinson's disease and epilepsy; gastrointestinal lesions; erythromelalgic or post-poliomyelitic pain, trigeminal or post-herpetic neuralgia; or as an anticonvulsant, analgesic or anxiolytic.

21. Use according to any one of claims 18 - 20, **characterised in that** an employed substituted 1-aminobutan-3-ol derivative as defined in any one of claims 9 to 16 is present as a pure diastereomer and/or enantiomer, as a racemate, or as a non-equimolar or equimolar mixture of the diastereomers and/or enantiomers.

22. Process for the production of a substituted 1-aminobutan-3-ol derivative according to any one of claims 1 to 8, in which a β-aminoketone of the formula **Ia** in which the radicals R¹ to R⁴, R⁶ and R⁷ have the meaning described in claim 1 for formula **I** 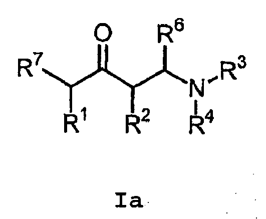 is reacted with an organometallic compound of the formula **III**
R⁵-Z **III**
in which Z denotes MgCl, MgBr, MgI or Li and R⁵ has the meaning described in claim 1 for formula **I**, to form a compound of the formula **I**.

## Revendications

1. Dérivés substitués de 1-aminobutane-3-ol, de formule générale I 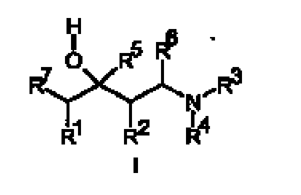 dans laquelle
R¹ et R² forment ensemble un noyau (CH₂)₂₋₉ qui peut être substitué le cas échéant avec un reste alkyle en C₁ à C₈, ramifié ou non ramifié, saturé ou non saturé, non substitué ou substitué une ou plusieurs fois ; ou avec un reste aryle, non substitué ou substitué une ou plusieurs fois,
R³ et R⁴ sont tous deux choisis indépendamment l'un de l'autre entre un reste alkyle en C₁ à C₆, ramifié ou non ramifié, saturé ou non saturé, non substitué ou substitué une ou plusieurs fois ; un reste cycloalkyle en C₃ à C₆, saturé ou non saturé, non substitué ou substitué une ou plusieurs fois ; un reste phényle, benzyle ou phénéthyle, non substitué ou substitué une ou plusieurs fois, ou bien
les restes R³ et R⁴ forment ensemble un noyau et représentent CH₂CH₂OCH₂CH₂, CH₂CH₂NR²²CH₂CH₂ ou (CH₂)₃₋₆,
où R²² est choisi entre H ; un reste alkyle en C₁ à C₁₀ ou cycloalkyle en C₃ à C₁₀, dont chacun est saturé ou non saturé, ramifié ou non ramifié, substitué une ou plusieurs fois ou non substitué ; un reste aryle ou hétéroaryle dont chacun est substitué une ou plusieurs fois ou non substitué ; ou un reste aryle, cycloalkyle en C₃ à C₁₀ ou hétéroaryle lié par l'intermédiaire d'un reliquat d'alkyle en C₁ à C₃ saturé ou non saturé, chacun étant substitué une ou plusieurs fois ou non substitué ;
R⁵ est choisi entre un reste alkyle en C₁ à C₁₀, saturé ou non saturé, ramifié ou non ramifié, substitué une ou plusieurs fois ou non substitué ; un reste cycloalkyle en C₃ à C₉, saturé ou non saturé ; un reste aryle, hétéroaryle, aryle lié par un radical alkyle en C₁ à C₃ saturé ou non saturé, cycloalkyle en C₃ à C₁₀ lié par l'intermédiaire d'un reliquat d'alkyle en C₁ à C₃ saturé ou non saturé, ou hétéroaryle lié par l'intermédiaire d'un reliquat d'alkyle en C₁ à C₃ saturé ou non saturé, tous les restes aryle, hétéroaryle et cycloalkyle pouvant dans chaque cas, indépendamment les uns des autres, être non substitués ou substitués une ou plusieurs fois avec des restes choisis indépendamment les uns des autres, entre
F, Cl, Br, I, OR¹⁸, SR¹⁸, SO₂R¹⁸, SO₂OR¹⁸, CN, COOR¹⁸, NR¹⁹R²⁰ ; alkyle en C₁ à C₁₀, saturé ou non saturé, ramifié ou non ramifié, substitué une ou plusieurs fois ou non substitué ; cycloalkyle en C₃ à C₉, saturé ou non saturé, non substitué ou substitué une ou plusieurs fois ; aryle ou hétéroaryle, chacun non substitué ou substitué une ou plusieurs fois ; ou aryle, cycloalkyle en C₃ à C₉ ou hétéroaryle lié par l'intermédiaire d'un radical alkyle en C₁ à C₃ saturé ou non saturé, chacun étant non substitué ou substitué une ou plusieurs fois ;
R¹⁸ étant choisi entre H ; alkyle en C₁ à C₁₀, saturé ou non saturé, ramifié ou non ramifié, non substitué ou substitué une ou plusieurs fois ; cycloalkyle en C₃ à C₉, saturé ou non saturé, non substitué ou substitué une ou plusieurs fois ; aryle ou hétéroaryle, non substitué ou substitué une ou plusieurs fois ; ou bien aryle, cycloalkyle en C₃ à C₉ ou hétéroaryle lié par l'intermédiaire d'un radical alkyle en C₁ à C₃ saturé ou non saturé, chacun étant non substitué ou substitué une ou plusieurs fois ;
R¹⁹ et R²⁰ sont choisis indépendamment l'un de l'autre entre H ; alkyle en C₁ à C₆, saturé ou non saturé, ramifié ou non ramifié, non substitué ou substitué une ou plusieurs fois ; cycloalkyle en C₃ à C₉, saturé ou non saturé, non substitué ou substitué une ou plusieurs fois ; aryle ou hétéroaryle, chacun non substitué ou substitué une ou plusieurs fois ; ou bien aryle, cycloalkyle en C₃ à C₉ ou hétéroaryle lié par l'intermédiaire d'un radical alkyle en C₁ à C₃ saturé ou non saturé, chacun étant non substitué ou substitué une ou plusieurs fois ;
ou bien R¹⁹ et R²⁰ forment ensemble CH₂CH₂OCH₂CH₂, CH₂CH₂NR²¹CH₂CH₂ ou (CH₂)₃₋₆,
R²¹ étant choisi entre H, alkyle en C₁ à C₁₀ saturé ou non saturé, ramifié ou non ramifié, non substitué ou substitué une ou plusieurs fois ;
R⁶ est choisi entre H ; aryle ou hétéroaryle, chacun non substitué ou substitué une ou plusieurs fois ; et
R⁷ est choisi entre halogène, CF₃, cycloalkyle en C₃ à C₉, saturé ou non saturé, non substitué ou substitué une ou plusieurs fois ; aryle, cycloalkyle en C₃ à C₉ ou hétéroaryle lié par l'intermédiaire d'un radical alkyle en C₁ à C₃ saturé ou non saturé, chacun non substitué ou substitué une ou plusieurs fois,
éventuellement sous forme de leurs racémates, de leurs stéréoisomères purs, en particulier leurs énantiomères ou diastéréoisomères ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels acceptables du point de vue physiologique, ou sous forme de leurs produits de solvatation, en particulier des hydrates.

2. Dérivés substitués de 1-aminobutane-3-ol suivant la revendication 1, **caractérisés en ce que**
R⁶ est choisi entre H ou un reste hétéroaryle ou avantageusement un reste de formule II 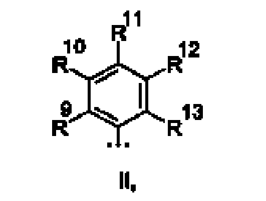 dans laquelle R⁹ à R¹³ sont choisis, indépendamment les uns des autres, chacun entre H, F, Cl, Br, I, CH₃, OH, OR₁₄, OCF₃, SR¹⁴, SO₂CH₃, SO₂CF₃ ; alkyle en C₁ à C₆, ramifié ou non ramifié, saturé ou non saturé, non substitué ou substitué une ou plusieurs fois ; phényle, non substitué ou substitué une ou plusieurs fois ; CN, COOR¹⁴, NO₂, ou bien
R⁹ et R¹⁰ ou R¹⁰ et R¹¹ forment ensemble un noyau OCH₂O ou OCH₂CH₂O, et
R¹⁴ est choisi entre alkyle en C₁ à C₆, ramifié ou non ramifié, saturé ou non saturé, non substitué ou substitué une ou plusieurs fois ; phényle, benzyle, phénéthyle ou thiophène, chacun non substitué ou substitué une ou plusieurs fois.

3. Dérivés substitués de 1-aminobutane-3-ol suivant l'une des revendications 1 ou 2, **caractérisés en ce que**
R¹ et R² forment ensemble un noyau (CH₂)₂₋₅ qui peut être substitué le cas échéant avec un reste alkyle en C₁ à C₆, ramifié ou non ramifié, saturé ou non saturé, non substitué ou substitué une ou plusieurs fois ; ou un reste phényle, non substitué ou substitué une ou plusieurs fois, ce noyau étant toutefois avantageusement non substitué.

4. Dérivés substitués de 1-aminobutane-3-ol suivant l'une des revendications 1 à 3, **caractérisés en ce que**
R³ et R⁴ sont choisis indépendamment l'un de l'autre, chacun entre alkyle en C₁ à C₆, ramifié ou non ramifié, saturé ou non saturé, non substitué ou substitué une ou plusieurs fois ; tous deux représentant avantageusement un reste CH₃,
ou bien les restes R³ et R⁴ forment ensemble un noyau et représentent CH₂CH₂NR²²CH₂CH₂ ou (CH₂)₃₋₆, et représentent l'ensemble en particulier (CH₂)₄₋₅ ou CH₂CH₂NR²²CH₂CH₂, où R²² est choisi entre H et un reste alkyle en C₁ à C₆, saturé, ramifié ou non ramifié et non substitué ; en particulier H ou CH₃.

5. Dérivés substitués de 1-aminobutane-3-ol suivant l'une des revendications 1 à 4, **caractérisés en ce que**
R⁵ est choisi entre alkyle en C₁ à C₆, saturé ou non saturé, ramifié ou non ramifié, substitué une ou plusieurs fois ou non substitué ; cycloalkyle en C₅ ou C₆, phényle, thiophényle, furyle, benzofurannyle, benzothiophényle, pyrrolyle, pyridinyle, pyrimidinyle, quinolinyle, isoquinolinyle, quinazolinyle, phényle lié par l'intermédiaire d'un radical alkyle en C₁ à C₃ saturé ou non saturé, cycloalkyle en C₅ ou C₆ lié par l'intermédiaire d'un radical alkyle en C₁ à C₃ substitué ou non substitué ou thiophényle, furyle, benzofurannyle, benzothiophényle, pyrrolyle, pyridinyle, pyrimidinyle, quinolinyle, isoquinolinyle, quinazolinyle, lié par l'intermédiaire d'un radical alkyle en C₁ à C₃ saturé ou non saturé, tous les restes aryle, hétéroaryle et cycloalkyle pouvant dans chaque cas, indépendamment les uns des autres, être non substitués ou substitués une ou plusieurs fois,
R⁵ étant avantageusement choisi entre un reste phényle ou thiophényle, non substitué ou substitué une ou plusieurs fois, avantageusement avec F, Cl, Br, I, OH, O-(alkyle en C₁ à C₄) , alkyle en C₁ à C₆, NH₂ et/ou SH ; ou phényle lié par l'intermédiaire d'un radical alkyle en C₁ à C₃ saturé ou non saturé, non substitué ou substitué une ou plusieurs fois, avantageusement avec F, Cl, Br, I, OH, O-(alkyle en C₁ à C₄), alkyle en C₁ à C₆, NH₂ et/ou SH,
en particulier
R⁵ est choisi entre phényle non substitué ou substitué une ou plusieurs fois, avantageusement avec F, Cl, Br, I, OH, OCH₃, OC₂H₅, OC₃H₇, SH, CH₃, C₂H₅, C₃H₇ et/ou C₄H₉.

6. Dérivés substitués de 1-aminobutane-3-ol suivant l'une des revendications 1 à 5, **caractérisés en ce que**
R⁷ est choisi entre cycloalkyle en C₅ à C₇, saturé ou non saturé, non substitué ou substitué une ou plusieurs fois ; avantageusement cyclohexyle ; phényle, non substitué ou substitué une ou plusieurs fois ; phényle lié par l'intermédiaire d'un radical alkyle en C₁ à C₃ saturé ou non saturé, dans chaque cas non substitué ou substitué une ou plusieurs fois ;
R⁷ est avantageusement choisi entre phényle, non substitué ou substitué une ou plusieurs fois ; phényle lié par l'intermédiaire d'un radical alkyle en C₁ à C₃ saturé ou non saturé, non substitué dans chaque cas ou substitué une ou plusieurs fois.

7. Dérivés substitués de 1-aminobutane-3-ol suivant l'une des revendications 1 à 6, **caractérisés en ce que** lorsque R⁵ et/ou R⁷ est choisi entre aryle, cycloalkyle en C₃ à C₉ ou hétéroaryle lié par l'intermédiaire d'un radical alkyle en C₁ à C₃ saturé ou non saturé, le radical alkyle en C₁ à C₃ par l'intermédiaire duquel le reste aryle, hétéroaryle ou cycloalkyle est lié, est choisi entre :
- CH₂-, -C₂H₄-, C₃H₆-, -C≡C-, -CH=CH-, -CH=CH-CH₂-, -CH₂-CH=CH-, -C=C-CH₂- ou -CH₂-C≡C-, avantageusement -CH₂-, -C₂H₄- ou -C≡C-.

8. Dérivés substitués de 1-aminobutane-3-ol suivant l'une des revendications 1 à 7, **caractérisés en ce qu'**ils sont choisis dans le groupe suivant :
• 2-benzyl-1-(2,4-dichlorobenzyl)-6-diméthylaminométhyl-cyclohexanol
• 2-benzyl-1-(3-chlorobenzyl)-6-diméthylaminométhyl-cyclohexanol
• 2-benzyl-6-diméthylaminométhyl-1-(2,5-diméthylbenzyl)-cyclohexanol
• 2-benzyl-1-(2-chloro-6-fluorobenzyl)-6-diméthylaminométhylcyclohexanol
• 2-benzyl-1-(4-chlorobenzyl)-6-diméthylaminométhyl-cyclohexanol
• 2-benzyl-6-diméthylaminométhyl-1-(3-trifluorométhylphényl)-cyclohexanol
• 2-benzyl-6-diméthylaminométhyl-1-(2-méthylbenzyl)-cyclohexanol
• 2-benzyl-6-diméthylaminométhyl-1-(2-méthoxyphényl)-cyclohexanol
• 2-benzyl-1-(4-chloro-3-trifluorométhylphényl)-6-diméthylaminométhylcyclohexanol
• 2-benzyl-1-(2-chlorobenzyl)-6-diméthylaminométhyl-cyclohexanol
• 2-benzyl-1-(3,5-dichlorophényl)-6-diméthylaminométhyl-cyclohexanol
• 2-benzyl-1-(3-chlorophényl)-6-diméthylaminométhyl-cyclohexanol
• 2-benzyl-6-diméthylaminométhyl-1-(3-fluorophényl)-cyclohexanol
• 2-benzyl-6-diméthylaminométhyl-1-(5-fluoro-2-méthoxyphényl)-cyclohexanol
• 2-benzyl-1-cyclohexylméthyl-6-diméthylaminométhyl-cyclohexanol
• 2-benzyl-6-diméthylaminométhyl-1-(4-méthoxyphényl)-cyclohexanol
• 2-benzyl-6-diméthylaminométhyl-1-p-tolyl-cyclohexanol
• 2-benzyl-6-diméthylaminométhyl-1-(3-phénylpropyl)-cyclohexanol
• 2-benzyl-6-diméthylaminométhyl-1-(3-méthoxyphényl)-cyclohexanol
• 2-benzyl-6-diméthylaminométhyl-1-thiophène-2-yl-cyclohexanol
• 2-benzyl-6-diméthylaminométhyl-1-phényléthynyl-cyclohexanol
• 2-benzyl-6-diméthylaminométhyl-1-(4-fluorophényl)-cyclohexanol
• 2-benzyl-6-diméthylaminométhyl-bicyclohexyl-1-ol
• 2-benzyl-6-diméthylaminométhyl-1-m-tolyl-cyclohexanol
• 2-benzyl-1-(4-tertiobutylphényl)-6-diméthylaminométhyl-cyclohexanol
• 2-benzyl-6-diméthylaminométhyl)-1-vinyl-cyclohexanol
• 2-benzyl-6-diméthylaminométhyl-1-o-tolyl-cyclohexanol
• 2-benzyl-6-diméthylaminométhyl-1-(4-fluoro-3-méthylphényl)-cyclohexanol
• 1,2-dibenzyl-6-diméthylaminométhyl-cyclohexanol
• 2-benzyl-1-(4-chlorophényl)-6-diméthylaminométhyl-cyclohexanol
• 2-benzyl-6-diméthylaminométhyl-1-phényl-cyclohexanol
• 2-diméthylaminométhyl-1-(2,5-diméthylphényl)-6-(3-méthoxyphényl)-cyclohexanol
• 1-cyclohexylméthyl-2-diméthylaminométhyl-6-(3-méthoxyphényl)-cyclohexanol
• 1-(2,4-dichlorobenzyl)-2-diméthylaminométhyl-6-(3-méthoxyphényl)-cyclohexanol
• 2-diméthylaminométhyl-6-(3-méthoxyphényl)-1-phényléthynylcyclohexanol
• 2-diméthylaminométhyl-6-(3-méthoxyphényl)-1-o-tolyl-cyclohexanol
• 1-(4-chlorophényl)-2-diméthylaminométhyl-6(3-méthoxyphényl)-cyclohexanol
• 2-diméthylaminométhyl-1-(3-fluorophényl)-6-(3-méthoxyphényl)-cyclohexanol
• 2-diméthylaminométhyl-1-(3-fluoro-4-méthoxyphényl)-6-(3-méthoxyphényl)-cyclohexanol
• 1-(3-chlorobenzyl)-2-diméthylaminométhyl-6-(3-méthoxyphényl)-cyclohexanol
• 2-diméthylaminométhyl-1-(3-méthoxybenzyl)-6-(3-méthoxyphényl)-cyclohexanol
• 1-(4-chloro-3-trifluorométhylphényl)-2-diméthylaminométhyl-6-(3-méthoxyphényl)-cyclohexanol
• 1-(3,5-dichlorobenzyl)-2-diméthylaminométhyl-6-(méthoxyphényl)-cyclohexanol
• 2-diméthylaminométhyl-6-(3-méthoxyphényl)-1-phényléthynylcyclohexanol
• 1-(4-chlorophényl)-2-diméthylaminométhyl-6-(3-méthoxyphényl)-cyclohexanol
• 2-diméthylaminométhyl-6-(3-méthoxyphényl)-1-o-tolyl-cyclohexanol
• 1-(3-chlorobenzyl)-2-diméthylaminométhyl-6-(3-méthoxyphényl)-cyclohexanol
• 2-diméthylaminométhyl-1-(4-fluoro-2-méthoxyphényl)-6-(3-méthoxyphényl)-cyclohexanol
• 2-diméthylaminométhyl-1-(2-méthoxyphényl)-6-(3-méthoxyphényl)-cyclohexanol
• 2-diméthylaminométhyl-1-(4-fluorobenzyl)-6-(3-méthoxyphényl)-cyclohexanol
• 1-(3-chlorophényl)-2-diméthylaminométhyl-6-(3-méthoxyphényl)-cyclohexanol
• 2-diméthylaminométhyl-6-(3-méthoxyphényl)-1-p-tolyl-cyclohexanol
• 2-diméthylaminométhyl-6-(3-méthoxyphényl)-1-(3-phénylpropyl)-cyclohexanol
• 2-diméthylaminométhyl-1,6-bis-(3-méthoxyphényl)-cyclohexanol
• 2-diméthylaminométhyl-6-(3-méthoxyphényl)-1-thiophène-2-yl-cyclohexanol
• 2-diméthylaminométhyl-6-(3-méthoxyphenyl)-1-phénéthyl-cyclohexanol
• 3-[3-diméthylaminométhyl-2-(4-fluorophényl)-2-hydroxycyclohexyl]-phénol
• 3-(3-diméthylaminométhyl-2-hydroxy-2-phényl-cyclohexyl)-phénol
• 3-[2-(4-tertiobutylphényl)-3-diméthylaminométhyl-2-hydroxycyclohexyl]-phénol
• 3-(3-diméthylaminométhyl-2-hydroxy-2-vinyl-cyclohexyl)-phénol
• 1-(4-chlorophényl)-2-diméthylaminométhyl-6-(3-méthoxyphényl)-cyclohexanol
• 3-diméthylaminométhyl-2-(3-méthoxyphényl)-bicyclohexyl-2-ol
• 2-benzyl-6-diméthylaminométhyl-1-(4-trifluorométhylphényl)-cyclohexanol
• 3-(2-benzyl-6-diméthylaminométhyl-1-hydroxy-cyclohexyl)-phénol
• 3-(2-tertiobutyl-6-diméthylaminométhyl-1-hydroxycyclohexyl)-phénol
sous forme de leurs stéréoisomères, en particulier leurs énantiomères ou diastéréoisomères.

9. Médicament contenant au moins un dérivé substitué de 1-aminobutane-3-ol de formule générale I 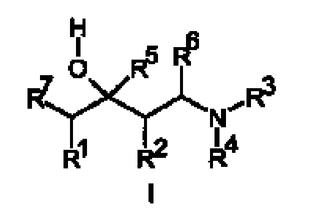 dans lequel
R¹ et R² forment ensemble un noyau (CH₂)₂₋₉ qui peut être substitué le cas échéant avec un reste alkyle en C₁ à C₈, ramifié ou non ramifié, saturé ou non saturé, non substitué ou substitué une ou plusieurs fois ; ou avec un reste aryle, non substitué ou substitué une ou plusieurs fois,
R³ et R⁴ sont tous deux choisis indépendamment l'un de l'autre entre un reste alkyle en C₁ à C₆, ramifié ou non ramifié, saturé ou non saturé, non substitué ou substitué une ou plusieurs fois ; un reste cycloalkyle en C₃ à C₆, saturé ou non saturé, non substitué ou substitué une ou plusieurs fois ; un reste phényle, benzyle ou phénéthyle, non substitué ou substitué une ou plusieurs fois, ou bien
les restes R³ et R⁴ forment ensemble un noyau et représentent CH₂CH₂OCH₂CH₂, CH₂CH₂NR²²CH₂CH₂ ou (CH₂)₃₋₆,
où R²² est choisi entre H ; un reste alkyle en C₁ à C₁₀ ou cycloalkyle en C₃ à C₁₀, dont chacun est saturé ou non saturé, ramifié ou non ramifié, substitué une ou plusieurs fois ou non substitué ; un reste aryle ou hétéroaryle dont chacun est substitué une ou plusieurs fois ou non substitué ; ou un reste aryle, cycloalkyle en C₃ à C₁₀ ou hétéroaryle lié par l'intermédiaire d'un reliquat d'alkyle en C₁ à C₃ saturé ou non saturé, chacun étant substitué une ou plusieurs fois ou non substitué ;
R⁵ est choisi entre un reste alkyle en C₁ à C₁₀, saturé ou non saturé, ramifié ou non ramifié, substitué une ou plusieurs fois ou non substitué ; un reste cycloalkyle en C₃ à C₉, saturé ou non saturé ; un reste aryle, hétéroaryle, aryle lié par un radical alkyle en C₁ à C₃ saturé ou non saturé, cycloalkyle en C₃ à C₁₀ lié par l'intermédiaire d'un reliquat d'alkyle en C₁ à C₃ saturé ou non saturé, ou hétéroaryle lié par l'intermédiaire d'un reliquat d'alkyle en C₁ à C₃ saturé ou non saturé, tous les restes aryle, hétéroaryle et cycloalkyle pouvant dans chaque cas, indépendamment les uns des autres, être non substitués ou substitués une ou plusieurs fois avec des restes choisis indépendamment les uns des autres, entre
F, Cl, Br, I, OR¹⁸, SR¹⁸, SO₂R¹⁸, SO₂OR¹⁸, CN, COOR¹⁸, NR¹⁹R²⁰ ; alkyle en C₁ à C₁₀, saturé ou non saturé, ramifié ou non ramifié, substitué une ou plusieurs fois ou non substitué ; cycloalkyle en C₃ à C₉, saturé ou non saturé, non substitué ou substitué une ou plusieurs fois ; aryle ou hétéroaryle, chacun non substitué ou substitué une ou plusieurs fois ; ou aryle, cycloalkyle en C₃ à C₉ ou hétéroaryle lié par l'intermédiaire d'un radical alkyle en C₁ à C₃ saturé ou non saturé, chacun étant non substitué ou substitué une ou plusieurs fois ;
R¹⁸ étant choisi entre H ; alkyle en C₁ à C₁₀, saturé ou non saturé, ramifié ou non ramifié, non substitué ou substitué une ou plusieurs fois ; cycloalkyle en C₃ à C₉, saturé ou non saturé, non substitué ou substitué une ou plusieurs fois ; aryle ou hétéroaryle, non substitué ou substitué une ou plusieurs fois ; ou bien aryle, cycloalkyle en C₃ à C₉ ou hétéroaryle lié par l'intermédiaire d'un radical alkyle en C₁ à C₃ saturé ou non saturé, chacun étant non substitué ou substitué une ou plusieurs fois ;
R¹⁹ et R²⁰ sont choisis indépendamment l'un de l'autre entre H ; alkyle en C₁ à C₆, saturé ou non saturé, ramifié ou non ramifié, non substitué ou substitué une ou plusieurs fois ; cycloalkyle en C₃ à C₉, saturé ou non saturé, non substitué ou substitué une ou plusieurs fois ; aryle ou hétéroaryle, chacun non substitué ou substitué une ou plusieurs fois ; ou bien aryle, cycloalkyle en C₃ à C₉ ou hétéroaryle lié par l'intermédiaire d'un radical alkyle en C₁ à C₃ saturé ou non saturé, chacun étant non substitué ou substitué une ou plusieurs fois ;
ou bien R¹⁹ et R²⁰ forment ensemble CH₂CH₂OCH₂CH₂, CH₂CH₂NR²¹CH₂CH₂ ou (CH₂)₃₋₆,
R²¹ étant choisi entre H, alkyle en C₁ à C₁₀ saturé ou non saturé, ramifié ou non ramifié, non substitué ou substitué une ou plusieurs fois ;
R⁶ est choisi entre H ; aryle ou hétéroaryle, chacun non substitué ou substitué une ou plusieurs fois ; et
R⁷ est choisi entre halogène, CF₃, alkyle en C₁ à C₆ ramifié ou non ramifié, saturé ou non saturé, non substitué ou substitué une ou plusieurs fois ; cycloalkyle en C₃ à C₉ saturé ou non saturé, non substitué ou substitué une ou plusieurs fois ; aryle, cycloalkyle en C₃ à C₉ ou hétéroaryle lié par l'intermédiaire d'un radical alkyle en C₁ à C₃ saturé ou non saturé, chacun non substitué ou substitué une ou plusieurs fois,
éventuellement sous forme de leurs racémates, de leurs stéréoisomères purs, en particulier leurs énantiomères ou diastéréoisomères ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels acceptables du point de vue physiologique, ou sous forme de leurs produits de solvatation, en particulier des hydrates,
ainsi que le cas échéant des additifs et/ou des substances auxiliaires convenables et/ou le cas échéant d'autres substances actives.

10. Médicament selon la revendication 9, **caractérisé en ce que**
R⁶ est choisi entre H ou un hétéroaryle ou avantageusement un reste de formule II 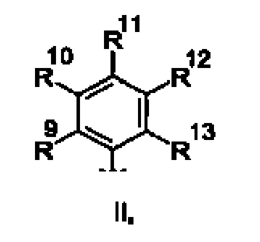 dans laquelle R⁹ à R¹³ sont choisis, indépendamment les uns des autres, chacun entre H, F, Cl, Br, I, CH₃, OH, OR¹⁴, OCF₃, SR¹⁴, SO₂CH₃, SO₂CF₃ ; alkyle en C₁ à C₆, ramifié ou non ramifié, saturé ou non saturé, non substitué ou substitué une ou plusieurs fois ; phényle, non substitué ou substitué une ou plusieurs fois ; CN, COOR¹⁴, NO₂, ou bien
R⁹ et R¹⁰ ou R¹⁰ et R¹¹ forment ensemble un noyau OCH₂O ou OCH₂CH₂O, et
R¹⁴ est choisi entre alkyle en C₁ à C₆, ramifié ou non ramifié, saturé ou non saturé, non substitué ou substitué une ou plusieurs fois ; phényle, benzyle, phénéthyle ou thiophène, chacun non substitué ou substitué une ou plusieurs fois.

11. Médicament selon la revendication 9 ou 10, **caractérisé en ce que**
R¹ et R² forment ensemble un noyau (CH₂)₂₋₅ qui peut être substitué le cas échéant avec un reste alkyle en C₁ à C₆, ramifié ou non ramifié, saturé ou non saturé, non substitué ou substitué une ou plusieurs fois ; ou un reste phényle, non substitué ou substitué une ou plusieurs fois, ce noyau étant toutefois avantageusement non substitué.

12. Médicament selon une ou plusieurs des revendications 9 à 11, **caractérisé en ce que**
R³ et R⁴ sont choisis indépendamment l'un de l'autre, chacun entre alkyle en C₁ à C₆, ramifié ou non ramifié, saturé ou non saturé, non substitué ou substitué une ou plusieurs fois ; tous deux représentant avantageusement un reste CH₃,
ou bien les restes R³ et R⁴ forment ensemble un noyau et représentent CH₂CH₂NR²²CH₂CH₂ ou (CH₂)₃₋₆, et représentent l'ensemble en particulier (CH₂)₄₋₅ ou CH₂CH₂NR²²CH₂CH₂, où R²² est choisi entre H et un reste alkyle en C₁ à C₆, saturé, ramifié ou non ramifié et non substitué ; en particulier H ou CH₃.

13. Médicament selon une ou plusieurs des revendications 9 à 12, **caractérisé en ce que**
R⁵ est choisi entre alkyle en C₁ à C₆, saturé ou non saturé, ramifié ou non ramifié, substitué une ou plusieurs fois ou non substitué ; cycloalkyle en C₅ ou C₆, phényle, thiophényle, furyle, benzofurannyle, benzothiophényle, pyrrolyle, pyridinyle, pyrimidinyle, quinolinyle, isoquinolinyle, quinazolinyle, phényle lié par l'intermédiaire d'un radical alkyle en C₁ à C₃ saturé ou non saturé, cycloalkyle en C₅ ou C₆ lié par l'intermédiaire d'un radical alkyle en C₁ à C₃ substitué ou non substitué ou thiophényle, furyle, benzofurannyle, benzothiophényle, pyrrolyle, pyridinyle, pyrimidinyle, quinolinyle, isoquinolinyle, quinazolinyle, lié par l'intermédiaire d'un radical alkyle en C₁ à C₃ saturé ou non saturé, tous les restes aryle, hétéroaryle et cycloalkyle pouvant dans chaque cas, indépendamment les uns des autres, être non substitués ou substitués une ou plusieurs fois,
R⁵ étant avantageusement choisi entre un reste phényle ou thiophényle, non substitué ou substitué une ou plusieurs fois, avantageusement avec F, Cl, Br, I, OH, O-(alkyle en C₁ à C₄), alkyle en C₁ à C₆, NH₂ et/ou SH ; ou phényle lié par l'intermédiaire d'un radical alkyle en C₁ à C₃ saturé ou non saturé, non substitué ou substitué une ou plusieurs fois, avantageusement avec F, Cl, Br, I, OH, O-(alkyle en C₁ à C₄), alkyle en C₁ à C₆, NH₂ et/ou SH,
en particulier
R⁵ est choisi entre phényle non substitué ou substitué une ou plusieurs fois, avantageusement avec F, Cl, Br, I, OH, OCH₃, OC₂H₅, OC₃H₇, SH, CH₃, C₂H₅, C₃H₇ et/ou C₄H₉.

14. Médicament selon une ou plusieurs des revendications 9 à 13**, caractérisé en ce que :**
R⁷ est choisi entre alkyle en C₁ à C₆, ramifié ou non ramifié, saturé ou non saturé, non substitué ou substitué une ou plusieurs fois ; cycloalkyle en C₅ à C₇, saturé ou non saturé, non substitué ou substitué une ou plusieurs fois, avantageusement cyclohexyle ; phényle, non substitué ou substitué une ou plusieurs fois ; phényle lié par l'intermédiaire d'un radical alkyle en C₁ à C₃ saturé ou non saturé, dans chaque cas non substitué ou substitué une ou plusieurs fois ;
R⁷ est avantageusement choisi entre phényle, non substitué ou substitué une ou plusieurs fois ; phényle lié par l'intermédiaire d'un radical alkyle en C₁ à C₃ saturé ou non saturé, non substitué dans chaque cas ou substitué une ou plusieurs fois.

15. Médicament selon une ou plusieurs des revendications 9 à 14, **caractérisé en ce que** lorsque R⁵ et/ou R⁷ est choisi entre aryle, cycloalkyle en C₃ à C₉ ou hétéroaryle lié par l'intermédiaire d'un radical alkyle en C₁ à C₃ saturé ou non saturé, le radical alkyle en C₁ à C₃ par l'intermédiaire duquel le reste aryle, hétéroaryle ou cycloalkyle est lié, est choisi entre :
- CH₂-, -C₂H₄-, C₃H₆-, -C≡C-, -CH=CH-, -CH=CH-CH₂-, -CH₂-CH=CH-, -C=C-CH₂- ou -CH₂-C≡C-, avantageusement -CH₂-, -C₂H₄- ou -C≡C-.

16. Médicament selon une ou plusieurs des revendications 9 à 15, **caractérisé en ce que** les dérivés substitués de 1-aminobutane-3-ol sont choisis dans le groupe suivant :
• 2-benzyl-1-(2,4-dichlorobenzyl)-6-diméthylaminométhylcyclohexanol
• 2-benzyl-1-(3-chlorobenzyl)-6-diméthylaminométhyl-cyclohexanol
• 2-benzyl-6-diméthylaminométhyl-1-(2,5-diméthylbenzyl)-cyclohexanol
• 2-benzyl-1-(2-chloro-6-fluorobenzyl)-6-diméthylaminométhylcyclohexanol
• 2-benzyl-1-(4-chlorobenzyl)-6-diméthylaminométhyl-cyclohexanol
• 2-benzyl-6-diméthylaminométhyl-1-(3-trifluorométhylphényl)-cyclohexanol
• 2-benzyl-6-diméthylaminométhyl-1-(2-méthylbenzyl)-cyclohexanol
• 2-benzyl-6-diméthylaminométhyl-1-(2-méthoxyphényl)-cyclohexanol
• 2-benzyl-1-(4-chloro-3-trifluorométhylphényl)-6-diméthylaminométhylcyclohexanol
• 2-benzyl-1-(2-chlorobenzyl)-6-diméthylaminométhyl-cyclohexanol
• 2-benzyl-1-(3,5-dichlorophényl)-6-diméthylaminométhyl-cyclohexanol
• 2-benzyl-1-(3-chlorophényl)-6-diméthylaminométhyl-cyclohexanol
• 2-benzyl-6-diméthylaminométhyl-1-(3-fluorophényl)-cyclohexanol
• 2-benzyl-6-diméthylaminométhyl-1-(5-fluoro-2-méthoxyphényl)-cyclohexanol
• 2-benzyl-1-cyclohexylméthyl-6-diméthylaminométhyl-cyclohexanol
• 2-benzyl-6-diméthylaminométhyl-1-(4-méthoxyphényl)-cyclohexanol
• 2-benzyl-6-diméthylaminométhyl-1-p-tolyl-cyclohexanol
• 2-benzyl-6-diméthylaminométhyl-1-(3-phénylpropyl)-cyclohexanol
• 2-benzyl-6-diméthylaminométhyl-1-(3-méthoxyphényl)-cyclohexanol
• 2-benzyl-6-diméthylaminométhyl-1-thiophène-2-yl-cyclohexanol
• 2-benzyl-6-diméthylaminométhyl-1-phényléthynyl-cyclohexanol
• 2-benzyl-6-diméthylaminométhyl-1-(4-fluorophényl)-cyclohexanol
• 2-benzyl-6-diméthylaminométhyl-bicyclohexyl-1-ol
• 2-benzyl-6-diméthylaminométhyl-1-m-tolyl-cyclohexanol
• 2-benzyl-1-(4-tertiobutylphényl)-6-diméthylaminométhyl-cyclohexanol
• 2-benzyl-6-diméthylaminométhyl)-1-vinyl-cyclohexanol
• 2-benzyl-6-diméthylaminométhyl-1-o-tolyl-cyclohexanol
• 2-benzyl-6-diméthylaminométhyl-1-(4-fluoro-3-méthylphényl)-cyclohexanol
• 1,2-dibenzyl-6-diméthylaminométhyl-cyclohexanol
• 2-benzyl-1-(4-chlorophényl)-6-diméthylaminométhyl-cyclohexanol
• 2-benzyl-6-diméthylaminométhyl-1-phényl-cyclohexanol
• 2-diméthylaminométhyl-1-(2,5-diméthylphényl)-6-(3-méthoxyphényl)-cyclohexanol
• 1-cyclohexylméthyl-2-diméthylaminométhyl-6-(3-méthoxyphényl)-cyclohexanol
• 1-(2,4-dichlorobenzyl)-2-diméthylaminométhyl-6-(3-méthoxyphényl)-cyclohexanol
• 2-diméthylaminométhyl-6-(3-méthoxyphényl)-1-phényléthynylcyclohexanol
• 2-diméthylaminométhyl-6-(3-méthoxyphényl)-1-o-tolyl-cyclohexanol
• 1-(4-chlorophényl)-2-diméthylaminométhyl-6(3-méthoxyphényl)-cyclohexanol
• 2-diméthylaminométhyl-1-(3-fluorophényl)-6-(3-méthoxyphényl)-cyclohexanol
• 2-diméthylaminométhyl-1-(3-fluoro-4-méthoxyphényl)-6-(3-méthoxyphényl)-cyclohexanol
• 1-(3-chlorobenzyl)-2-diméthylaminométhyl-6-(3-méthoxyphényl)-cyclohexanol
• 2-diméthylaminométhyl-1-(3-méthoxybenzyl)-6-(3-méthoxyphényl)-cyclohexanol
• 1-(4-chloro-3-trifluorométhylphényl)-2-diméthylaminométhyl-6-(3-méthoxyphényl)-cyclohexanol
• 1-(3,5-dichlorobenzyl)-2-diméthylaminométhyl-6-(méthoxyphényl)-cyclohexanol
• 2-diméthylaminométhyl-6-(3-méthoxyphényl)-1-phényléthynylcyclohexanol
• 1-(4-chlorophényl)-2-diméthylaminométhyl-6-(3-méthoxyphényl)-cyclohexanol
• 2-diméthylaminométhyl-6-(3-méthoxyphényl)-1-o-tolyl-cyclohexanol
• 1-(3-chlorobenzyl)-2-diméthylaminométhyl-6-méthyl-cyclohexanol
• 2-diméthylaminométhyl-1-(2,5-diméthylbenzyl)-6-méthyl-cyclohexanol
• 1-(2-chloro-6-fluorobenzyl)-2-diméthylaminométhyl-6-méthylcyclohexanol
• 1-(4-chlorobenzyl)-2-diméthylaminométhyl-6-méthyl-cyclohexanol
• 2-diméthylaminométhyl-6-méthyl-1-(3-méthylbenzyl)-cyclohexanol
• 2-diméthylaminométhyl-6-méthyl-1-(3-trifluorométhylphényl)-cyclohexanol
• 1-(2-chloro-3-fluorophényl)-2-diméthylaminométhyl-6-méthylcyclohexanol
• 2-diméthylaminométhyl-6-méthyl-1-(2-méthylbenzyl)-cyclohexanol
• 2-diméthylaminométhyl-1-(2-méthoxyphényl)-6-méthyl-cyclohexanol
• 2-diméthylaminométhyl-1-(3-fluorobenzyl)-6-méthyl-cyclohexanol
• 2-diméthylaminométhyl-1-(4-fluorobenzyl)-6-méthyl-cyclohexanol
• 1-(2-chlorobenzyl)-2-diméthylaminométhyl-6-méthyl-cyclohexanol
• 1-(3,5-dichlorophényl)-2-diméthylaminométhyl-6-méthyl-cyclohexanol
• 1-(3-chlorophényl)-2-diméthylaminométhyl-6-méthyl-cyclohexanol
• 2-diméthylaminométhyl-1-(3-fluorophényl)-6-méthyl-cyclohexanol
• 2-diméthylaminométhyl-1-(3-fluoro-4-méthoxyphényl)-6-méthylcyclohexanol
• 2-diméthylaminométhyl-1-(4-méthoxyphényl)-6-méthyl-cyclohexanol
• 2-diméthylaminométhyl-6-méthyl-1-p-tolyl-cyclohexanol
• 2-diméthylaminométhyl-6-méthyl-1-(3-phénylpropyl)-cyclohexanol
• 2-diméthylaminométhyl-1-(3-méthoxyphényl)-6-méthyl-cyclohexanol
• 2-diméthylaminométhyl-6-méthyl-1-thiophène-2-yl-cyclohexanol
• 2-diméthylaminométhyl-6-méthyl-1-phényléthynyl-cyclohexanol
• 2-diméthylaminométhyl-6-méthyl-1-phénéthyl-cyclohexanol
• 2-diméthylaminométhyl-1-(4-fluorophényl)-6-méthyl-cyclohexanol
• 1-(3-chlorobenzyl)-2-diméthylaminométhyl-6-(3-méthoxyphényl)-cyclohexanol
• 2-diméthylaminométhyl-1-(4-fluoro-2-méthoxyphényl)-6-(3-méthoxyphényl)-cyclohexanol
• 2-diméthylaminométhyl-1-(2-méthoxyphényl)-6-(3-méthoxyphényl)-cyclohexanol
• 2-diméthylaminométhyl-1-(4-fluorobenzyl)-6-(3-méthoxyphényl)-cyclohexanol
• 1-(3-chlorophényl)-2-diméthylaminométhyl-6-(3-méthoxyphényl)-cyclohexanol
• 2-diméthylaminométhyl-6-(3-méthoxyphényl)-1-p-tolyl-cyclohexanol
• 2-diméthylaminométhyl-6-(3-méthoxyphényl)-1-(3-phénylpropyl)-cyclohexanol
• 2-diméthylaminométhyl-1,6-bis-(3-méthoxyphényl)-cyclohexanol
• 2-diméthylaminométhyl-6-(3-méthoxyphényl)-1-thiophène-2-ylcyclohexanol
• 2-diméthylaminométhyl-6-(3-méthoxyphenyl)-1-phénéthyl-cyclohexanol
• 3-[3-diméthylaminométhyl-2-(4-fluorophényl)-2-hydroxycyclohexyl]-phénol
• 3-(3-diméthylaminométhyl-2-hydroxy-2-phényl-cyclohexyl)-phénol
• 3-[2-(4-tertiobutylphényl)-3-diméthylaminométhyl-2-hydroxycyclohexyl]-phénol
• 3-(3-diméthylaminométhyl-2-hydroxy-2-vinyl-cyclohexyl)-phénol
• 1-(4-chlorophényl)-2-diméthylaminométhyl-6-(3-méthoxyphényl)-cyclohexanol
• 3-diméthylaminométhyl-2-(3-méthoxyphényl)-bicyclohexyl-2-ol
• 2-benzyl-6-diméthylaminométhyl-1-(4-trifluorométhylphényl)-cyclohexanol
• 3-(2-benzyl-6-diméthylaminométhyl-1-hydroxy-cyclohexyl)-phénol
• 3-(2-tertiobutyl-6-diméthylaminométhyl-1-hydroxycyclohexyl)-phénol
éventuellement sous forme de leur racémates, de leurs stéréoisomères, en particulier leurs énantiomères ou diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels acceptables du point de vue physiologique, ou sous forme de leurs produits de solvatation, en particulier des hydrates, en particulier des chlorhydrates.

17. Médicament suivant l'une des revendications 9 à 16, **caractérisé en ce qu'**un dérivé substitué de 1-aminobutane-3-ol suivant l'une des revendications 1 à 8, qui est contenu est présent sous forme de diastéréoisomère et/d'énantiomère pur, comme racémate ou comme mélange non équimolaire ou équimolaire des diastéréoisomères et/ou des énantiomères.

18. Utilisation d'un dérivé substitué de 1-aminobutane-3-ol suivant l'une des revendications 9 à 16 pour la préparation d'un médicament destiné au traitement de la douleur, en particulier de douleur aiguë, névropathique ou chronique.

19. Utilisation d'un dérivé substitué de 1-aminobutane-3-ol suivant l'une des revendications 9 à 16 pour la préparation d'un médicament destiné au traitement de la migraine, de l'hyperalgésie et de l'allodynie, en particulier de l'hyperalgésie thermique, de l'hyperalgésie et de l'allodynie mécanique, et de l'allodynie liée au froid, ou de douleur inflammatoire ou post-opératoire.

20. Utilisation d'un dérivé substitué de 1-aminobutane-3-ol suivant l'une des revendications 9 à 16 pour la préparation d'un médicament destiné au traitement de l'épilepsie, des bouffées de chaleur, des troubles post-ménopausiques, de la sclérose latérale amyotrophique (ALS), de l'algodystrophie sympathique réflexe (RSD), de la paralysie spastique, du Restless Leg Syndrome, du nystagmus acquis ; de troubles psychiatriques et neuropathologiques tels que troubles bipolaires, anxiété, crises de peur panique, variations de l'humeur, psychose maniaque, dépressions, psychose maniaco-dépressive ; de la neuropathie diabétique douloureuse, de symptômes et douleurs liées à la sclérose en plaques ou à la maladie de Parkinson, de maladies neurodégénératives telles que la maladie d'Alzheimer, la maladie de Huntington, la maladie de Parkinson et l'épilepsie ; d'une lésion gastrointestinale ; de douleur érythromélalgique ou post-poliomyélitique, de névralgie faciale ou post-herpétique ; ou comme anticonvulsif, analgésique ou anxiolytique.

21. Utilisation suivant l'une des revendications 18 à 20, **caractérisée en ce qu'**un dérivé substitué de 1-aminobutane-3-ol utilisé suivant l'une des revendications 9 à 16 est présent sous forme pure de diastéréoisomère et/ou d'énantiomère, comme racémate ou comme mélange non équimolaire ou équimolaire des diastéréoisomères et/ou des énantiomères.

22. Procédé de production d'un dérivé substitué de 1-aminobutane-3-ol suivant l'une des revendications 1 à 8, dans lequel une β-aminocétone de formule la 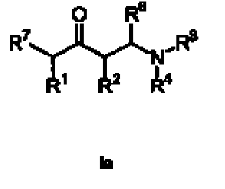 dans laquelle les restes R¹ à R⁴, R⁶ et R⁷ ont la définition indiquée pour la formule I dans la revendication 1,
est amenée à réagir avec un composé organométallique de formule III
R⁵-Z III
dans laquelle Z représente MgCl, MgBr, MgI ou Li et R⁵ a la définition donnée dans la revendication 1 pour la formule I, pour former un composé de formule I.
